# EUROPEAN PATENT SPECIFICATION

(11) **EP 1 278 540 B1**
(45) Date of publication and mention of the grant of the patent: **14.05.2008**
(21) Application number: 01945064.2
(22) Date of filing: 30.04.2001
(51) Int. Cl.: A61K 39/00, A61K 39/395, C07K 16/24, C07K 14/54, C07K 14/81, A61P 9/10, A61P 7/02

(54) **USE OF IL-18 INHIBITORS FOR THE TREATMENT AND/OR PREVENTION OF ATHEROSCLEROSIS**
VERWENDUNG VON IL-18 INHIBITOREN ZUR BEHANDLUNG UND/ODER PRÄVENTION VON ATHEROSKLEROSE
UTILISATION D'INHIBITEURS IL-18 POUR LE TRAITEMENT ET/OU LA PREVENTION DE L'ATHEROSCLEROSE

(30) Priority: 05.05.2000 EP 00109606
(43) Date of publication of application: 29.01.2003
(73) Proprietor: Laboratoires Serono SA, 1267 Coinsins, Vaud (CH); INSTITUT NATIONAL DE LA SANTE ET DE LA RECHERCHE MEDICALE (INSERM), 75654 Paris Cédex 13 (FR)
(72) Inventor: CHVATCHKO, Yolande, CH-1232 Confignon (CH); TEDGUI, Alain, F-75001 Paris (FR); MALLAT, Ziad, F-78580 Herbeville (FR)
(74) Representative: Merck Serono International S.A. Intellectual Property
(86) International application number: PCT/EP2001/004843
(87) International publication number: WO 2001/085201

(56) References cited:
- EP-A- 0 974 600
- WO-A-99/09063
- US-A- 5 877 197
- US-A- 5 985 863
- AIZAWA Y ET AL: "Cloning and expression of interleukin-18 binding protein" FEBS LETTERS, ELSEVIER SCIENCE PUBLISHERS, AMSTERDAM, NL, vol. 445, no. 2-3, 26 February 1999 (1999-02-26), pages 338-342, XP004259285 ISSN: 0014-5793
- YOUNG JAMES L ET AL: "The serpin proteinase inhibitor 9 is an endogenous inhibitor of interleukin 1beta-converting enzyme (caspase-1) activity in human vascular smooth muscle cells." JOURNAL OF EXPERIMENTAL MEDICINE, vol. 191, no. 9, 1 May 2000 (2000-05-01), pages 1535-1544, XP000946391 ISSN: 0022-1007
- KOBAYASHI ISAO ET AL: "Interleukin-18 modulates viral myocarditis." CIRCULATION, vol. 98, no. 17 SUPPL., 27 October 1998 (1998-10-27), page I297 XP001042302 71st Scientific Sessions of the American Heart Association;Dallas, Texas, USA; November 8-11, 1998 ISSN: 0009-7322
- SETA Y ET AL: "Interleukin 18 in acute myocardial infarction" HEART, BMJ, LONDON,, GB, vol. 84, no. 6, December 2000 (2000-12), page 668 XP001002516 ISSN: 1355-6037
- POMERANTZ B J ET AL: "Inhibition of caspase 1 reduces human myocardial ischemic dysfunction via inhibition of IL-18 and IL-1beta" PROCEEDINGS OF THE NATIONAL ACADEMY OF SCIENCES OF USA, NATIONAL ACADEMY OF SCIENCE. WASHINGTON, US, vol. 98, no. 5, 27 February 2001 (2001-02-27), pages 2871-2876, XP002168538 ISSN: 0027-8424
- AFFLECK D G ET AL: "Interleukin-18 production following murine cardiac transplantation:correlation with histologic rejection and the induction of IFN-gamma" JOURNAL OF INTERFERON AND CYTOKINE RESEARCH, MARY ANN LIEBERT, NEW YORK, NY, US, vol. 21, no. 1, January 2001 (2001-01), pages 1-9, XP001002506 ISSN: 1079-9907
- KANDA T ET AL: "Effect of interleukin-18 on viral myocarditis: enhancement of interferon-gamma and natural killer cell activity" JOURNAL OF MOLECULAR AND CELLULAR CARDIOLOGY, XX, XX, vol. 32, no. 12, December 2000 (2000-12), pages 2163-2171, XP001002525 ISSN: 0022-2828
- MALLAT ZIAD ET AL: "Expression of interleukin-18 in human atherosclerotic plaques and relation to plaque instability." CIRCULATION, vol. 104, no. 14, 2 October 2001 (2001-10-02), pages 1598-1603, XP001042303 ISSN: 0009-7322

### SEQUENCE LISTING

<110> Applied Research Systems ARS Holding N.V.
<120> Use of IL-18 inhibitors for the treatment and/or prevention of atherosclerosis
<130> 443 WO
<140> PCT/EP01/04843
   <141> 2001-04-30
<150> EP 00109606.4
   <151> 2000-05-05
<160> 14
<170> PatentIn version 3.1
<210> 1
   <211> 20
   <212> DNA
   <213> Homo sapiens
<220>
   <221> IL- 18 reverse primer
   <222> (1)..(20)
   <223>
<400> 1
   gcgtcactac actcagctaa
   20
<210> 2
   <211> 20
   <212> DNA
   <213> Homo sapiens
<220>
   <221> IL-18 forward primer
   <222> (1)..(20)
   <223>
<400> 2
   gcctagaggt atggctgtaa
   20
<210> 3
   <211> 20
   <212> DNA
   <213> Homo sapiens
<220>
   <221> IL-18BP forward primer
   <222> (1)..(20)
   <223>
<400> 3
   acctgtctac ctggagtgaa
   20
<210> 4
   <211> 20
   <212> DNA
   <213> Homo sapiens
<220>
   <221> IL-18BP reverse primer
   <222> (1)..(20)
   <223>
<400> 4
   gcacgaagat aggaagtctg
   20
<210> 5
   <211> 24
   <212> DNA
   <213> Homo sapiens
<220>
   <221> beta actin reverse primer
   <222> (1)..(24)
   <223>
<400> 5
   ggaggagcaa tgatcttgat cttc
   24
<210> 6
   <211> 24
   <212> DNA
   <213> Homo sapiens
<220>
   <221> beta actin forward primer
   <222> (1)..(24)
   <223>
<400> 6
   gctcaccatg gatgatgata tcgc
   24
<210> 7
   <211> 18
   <212> DNA
   <213> Homo sapiens
<220>
   <221> IL-18 reverse primer 2
   <222> (1) .. (18)
   <223>
<400> 7
   cagccgcttt agcagcca
   18
<210> 8
   <211> 21
   <212> DNA
   <213> Homo sapiens
<220>
   <221> IL-18 forward primer 2
   <222> (1)..(21)
   <223>
<400> 8
   caaggaattg tctcccagtg c
   21
<210> 9
   <211> 19
   <212> DNA
   <213> Homo sapiens
<220>
   <221> IL-18BP reverse primer 2
   <222> (1)..(19)
   <223>
<400> 9
   aaccaggctt gagcgttcc
   19
<210> 10
   <211> 24
   <212> DNA
   <213> Homo sapiens
<220>
   <221> IL-18BP forward primer 2
   <222> (1)..(24)
   <223>
<400> 10
   tcccatgtct ctgctcattt agtc
   24
<210> 11
   <211> 22
   <212> DNA
   <213> Homo sapiens
<220>
   <221> GAPDH reverse primer
   <222> (1)..(22)
   <223>
<400> 11
   gatgggattt ccattgatga ca
   22
<210> 12
   <211> 18
   <212> DNA
   <213> Homo sapiens
<220>
   <221> GAPDH forward primer
   <222> (1)..(18)
   <223>
<400> 12
   ccacccatgg caaattcc
   18
<210> 13
   <211> 21
   <212> DNA
   <213> Homo sapiens
<220>
   <221> intron GAPDH reverse primer
   <222> (1) .. (21)
   <223>
<400> 13
   cctagtccca gggctttgat t
   21
<210> 14
   <211> 22
   <212> DNA
   <213> Homo sapiens
<220>
   <221> intron GAPDH forward primer
   <222> (1) .. (22)
   <223>
<400> 14
   ctgtgctccc actcctgatt tc
   22

## Description

### FIELD OF THE INVENTION

The present invention is in the field of vascular diseases. More specifically, the invention relates to the use of IL-18 inhibitors for treatment and/or prevention of atherosclerosis.

### BACKGROUND OF THE INVENTION

Atherosclerosis is the commonest and most important vascular disease, but many other vascular disorders are recognised. Atherosclerosis mainly affects large and medium-sized arteries, and its lesions comprise fatty streaks, fobrolytic plaquest and complicated lesions. Atherosclerosis is a chronic inflammatory disease of the arterial wall characterized by progressive accumulation of lipids, like cholesterol, cells, like macrophages, T lymphocytes or smooth muscle cells, and extracellular matrix (1). Larger accumulations are called atheromas or plaques, which often contain calcium. The fatty tissue can erode the wall of the artery, diminish the elasticity of the artery, and interfere with the blood flow. Eventually, clots may form around the plaque deposits, further interfering with blood flow, which may lead to a total occlusion of the blood vessel. Usually, atherosclerosis is associated with increased levels of LDL-cholesterol, Lp(a) fibrinogen and factor VII, as well as reduced levels of HDL-cholesterol. Risk factors include increasing age, male gender, smoking, diabetes, obesity, high blood cholesterol, a diet high in fats, and having a personal or family historyof heart disease. It is the major cause of organ ischemia like e.g. myocardial infarction.

Atheroma is the commonest lesion in arteries, which may be further complicated by thrombo-embolism. Atheromatous plaques often narrow the lumen of arteries causing ischemia and sometimes atrophy of tissues in the hypoperfused territory. Serious consequences include the symptom of angina due to myocardial ischemia, heart failure due to ischemia or non-ischemic events, and hypertension due to renal artery narrowing and hypoperfusion of a kidney which responds physiologically by increased renin secretion.

Sometimes atherosclerosis and arteriosclerosis are referred to as separate pathological conditions, and in this case, atherosclerosis is defined as implying hardening (sclerosis) or loss of elasticity of arteries due specifically to atheroma, whilst arteriosclerosis is hardening or loss of elasticity of arteries from any cause.

Complications or consequences of atherosclerosis include coronary artery disease (atherosclerosis of the coronary arteries), deficiency of blood supply due to obstruction (ischemia/angina), acute MI (myocardial infarction, heart attack), transient ischemic attack (TIA) or stroke, and damage to blood vessels, muscles, or body organs

Aneurysms, which are permanent, abonormal dilatations of blood vessels, are also common consequences of atherosclerosis. Atherosclerotic abdominal aortic aneurysms commonly develop in elderly patients. They may rupture into the retroperitoneal space. In atherosclerotic aneurysms, there is usually a pronounced loss of elastic tissue and fibrosis of the media, mainly due to ischemia of the muscle of the aortic media, followed by release of macrophage enzymes causing fragmentation of elastic fibres.

Medications recommended for treatment or prevention of atherosclerosis include reduction of blood fats/cholesterol. In particular, LDL-cholesterol lowering therapy is widely used. At present statins, are specific inhibitors of HMG CoA reductase, are most widely used. Further fat lowering agents comprise medications such as cholestyramine, colestipol, nicotinic acid, gemfibrozil, probucol, lovastatin, and others.

Another approach is to minimise the risk of thrombus formation on established atheromatous lesions. Aspirin, which seems to be a specific inhibitor ofthromboxane A2 mediated platelet aggregation, or anticoagulants may be used to reduce risk of clot formation.

Percutaneous "balloon angioplasty" uses a balloon-tipped catheter to flatten plaque and increase blood flow past the occlusion. The technique is similar to that used to open the arteries of the heart, but it can be applied to many other arteries in the body. Coronary artery stenoses are bypassed with segments of saphenous vein sewn into the proximal aorta or by dissecting the internal mammary artery from the chest wall and anastomosing its distal end to an artery on the anterior surface of the heart.

Surgical removal of deposits (endarterectomy) may be recommended in some cases (example: carotid endarterectomy).

However, the major recommendation remains to treat or control risk factors, like keeping low-fat, low-cholesterol, and low-salt diet and follow the health care provider's recommendations for treatment and control of hypertension, diabetes, and other diseases, reduction of body weight, and stop smoking, as well as regular exercise to improve the fitness of the heart and circulation.

The inflammatory process is involved throughout the different stages of atherosclerosis (1). Endothelial activation, by various factors including low shear stress, modified lipoproteins and pro-inflammatory cytokines, is thought to be the first step in atherosclerosis and is under inflammatory control (1). Many recent studies have shown that interactions between vascular and inflammatory cells are crucial in atherogenesis (1). Particularly, inhibition of defined pro-inflammatory pathways reduced the development of atherosclerosis (1).

Inflammation also plays a major role in atherosclerotic plaque disruption and thrombosis (2-5), and therefore influences the occurrence of acute ischemic syndromes and their related mortality (6). Indeed, severe clinical manifestations of atherosclerosis, including infarctions of the heart, brain and any other organs affected by atherosclerosis, are mainly due to vessel lumen occlusion by a thrombus formed on the contact of a disrupted atherosclerotic plaque (3, 4). Pathological studies have shown that vulnerable or unstable plaques, i.e., plaques prone to rupture or having ruptured, greatly differ in cell and matrix composition compared with stable plaques, not prone to rupture (7). The vulnerable plaques are rich in inflammatory cells (macrophages and T lymphocytes), contain a thrombogenic lipid core and are characterised by a thin fibrous cap with a substantial loss in extracellular matrix (7).

Decreased collagen synthesis, mediated by the pro-inflammatory cytokine IFNγ, and increased activity of macrophage-derived matrix degrading metalloproteinases are responsible for fibrous cap thinning and fragility (7). Rupture of the fragile fibrous cap exposes the highly thrombogenic lipid core to the circulating blood and results in occlusive thrombus formation (1, 7). Therefore, the density of inflammatory cells in a given atherosclerotic lesion is considered to be a good indicator of its instability.

The clinical prognosis of a patient with atherosclerosis depends only in part on the size of the lesions (19;20). It is now widely recognised that the quality (plaque composition), rather than the size, of the lesion could be an even better predictor of the occurrence of ischemic events. Indeed, severe clinical manifestations of atherosclerosis (infarctions of the heart and brain) are mainly due to vessel lumen occlusion by a thrombus formed at the contact of a disrupted atherosclerotic plaque (19). Pathological studies have shown that vulnerable or unstable plaques, that are prone to rupture or have ruptured, are rich in inflammatory cells and exhibit a substantial loss in smooth muscle cell and collagen content (20, 21). Moreover, such plaques show significant increase in apoptotic cell death leading to the formation of a highly thrombogenic lipid core (13, 22).

Pro-inflammatory cytokines are involved in inflammation. The cytokine interleukin 18 (IL-18) was initially described as an interferon-γ (IFN-γ) inducing factor (8). It is an early signal in the development of T-lymphocyte helper cell type 1 (Th1) responses. IL-18 acts together with IL-12, IL-2, antigens, mitogens, and possibly further factors, to induce the production of IFN-γ. IL-18 also enhances the production of GM-CSF and IL-2, potentiates anti-CD3 induced T cell proliferation, and increases Fas-mediated killing of natural killer cells. Mature IL-18 is produced from its precursor by the IL-1β converting enzyme (ICE, caspase-1). The IL-18 receptor consists of at least two components, cooperating in ligand binding. High- and low-affinity binding sites for IL-18 were found in murine IL-12 stimulated T cells (9), suggesting a multiple chain receptor complex. Two receptor subunits have been identified so far, both belonging to the IL-1 receptor family (10). The signal transduction of IL-18 involves activation of NF-κB (11).

Recently, a soluble protein having a high affinity for IL-18 has been isolated from human urine, and the human and mouse cDNAs as well as the human gene were cloned (12; WO 99/09063). The protein has been designated IL-18 binding protein (IL-18BP).

IL18BP is not the extracellular domain of one of the known IL18 receptors, but a secreted, naturally circulating protein. It belongs to a novel family of secreted protein, further including several Poxvirus-encoded proteins (12). IL18BP is constitutively expressed in the spleen (12). Urinary as well as recombinant IL18BP specifically bind IL-18 with a high affinity and modulate the biological affinity of IL-18.

The IL18BP gene has been localised to the human chromosome 11q13, and no exon coding for a transmembrane domain was found in an 8.3kb genomic sequence. Four splice variants or isoforms of IL18BP were found in humans, and designated IL18BP a, b, c and d, all sharing the same N-terminus and differing in the C-terminus (12).

Four human and two mouse isoforms of IL-18BP, resulting from mRNA splicing and found in various cDNA libraries and have been expressed, purified, and assessed for binding and neutralization of IL-18 biological activities (23). Human IL-18BPisoform a (IL-18BPa) exhibited the greatest affinity for IL-18 with a rapid on-rate, a slow off-rate, and a dissociation constant (K(d)) of 399 pM. IL-18BPc shares the Ig domain of IL-18BPa except for the 29 C-terminal amino acids; the K(d) of IL-l8BPc is 10-fold less (2.94 nM). Nevertheless, IL-18BPa and IL-18BPc neutralize IL-18 >95% at a molar excess of two. IL-1 8BPb and IL-18BPd isoforms lack a complete Ig domain and lack the ability to bind or neutralize IL-18. Murine IL-18BPc and IL-18BPd isoforms, possessing the identical Ig domain, also neutralize >95% murine IL-18 at a molar excess of two. However, murine IL-18BPd, which shares a common C-terminal motif with human IL-18BPa, also neutralizes human IL-18. Molecular modelling identified a large mixed electrostatic and hydrophobic binding site in the Ig domain of IL-18BP, which could, account for its high affinity binding to the ligand (23).

### SUMMARY OF THE INVENTION ,

The invention is based on the finding that an inhibitor of IL-18 had a pronounced beneficial effect on plaque development, plaque progression and plaque stability in a murine model of atherosclerosis. The inhibitor of IL-18 not only prevented lesion formation in the thoracic aorta, but also induced a switch toward a stable plaque phenotype in already established atherosclerotic plaques.

Therefore, the invention relates to the use of an IL-18 inhibitor as defined in the claims for the manufacture of a medicament for the prevention and/or treatment of atherosclerosis. The invention further relates to methods of treatment for a gene therapeutic approach of treating and/or preventing atherosclerosis.

### BRIEF DESPRIPTION OF THE DRAWINGS

Figure 1 shows a histogram depicting the percentage of survival of human umbilical vein endothelial cells after incubation with oxidised lipoproteins alone, or incubation with a combination of oxidised lipoproteins and an IL-18 antibody or IL18BP, respectively.

Figure 2 shows a Western Blot performed on protein extracts from atherosclerotic arteries in comparison to control arteries. In the Western Blot, antibodies directed against IL-18BP (hIL18BP), IL-18 receptor a subunit (hIL18Rα), IL-18 (hIL18) and Caspase-1 (Caspase-1 p10) were used.

Figure 3 shows an ethidium bromide stained agarose gel showing the result of a RT-PCR analysis for IL-18 and IL-18BP mRNA in cells of the atherosclerotic plaque.

Figure 4 Representative RT-PCR results for IL-18BP and IL-18 in atherosclerotic plaques in comparison to hα-actin (control) expression in symptomatic and asymptomatic plaques.

Figure 5 shows the map of the expression vector used for intramuscular electrotransfer in mice.

Figure 6 shows a histogram depicting the lipid staining area in atherosclerotic arteries. Quantitative computer-assisted image analysis of lipid deposition. Data represent mean values with s.e.m. (n = 19 for empty plasmid, n = 14 for IL-18BP plasmid). Quadruple asterisks indicate P < 0.0001.

Figure 7 shows a histogram depicting the aortic sinus lesion area after IL-18BP-treatment as compared to control (empty plasmid). Quantitative computer-assisted image analysis of lesion area. Data represent mean values with s.e.m. (n = 19 for empty plasmid, n = 14 for IL-18BP plasmid). Double asterisks indicate P < 0.01.

Figure 8 shows the effect of IL-18BP treatment on lesion inflammatory cell content. Quantitative computer-assisted image analysis was used to determine the percentage of macrophage-positive areas (black bars) and the number of infiltrating T lymphocytes per mm² (grey bars) in aortic sinus lesions of control (n = 12 for macrophage staining, n = 15 for T lymphocyte staining) or IL-18BP treated mice (n = 13 for macrophages, n = 12 for T lymphocytes). Data represent mean values with s.e.m. Triple asterisks indicate P < 0.005; and quadruple asterisks indicate P < 0.0001.

Figure 9 shows the effect of IL-18BP treatment on lesion smooth muscle cell and collagen content. Quantitative computer-assisted image analysis was used to determine the percentage of smooth muscle cell-positive areas (black bars) and collagen accumulation (grey bars) in aortic sinus lesions of control (n = 6 for smooth muscle cells, n = 11 for collagen) and IL-18BP treated mice (n = 6 for smooth muscle cells, n = 13 for collagen). Data represent mean values with s.e.m. Single asterisk indicates P < 0.05; and double asterisks indicate P < 0.01.

### DESCRIPTION OF THE INVENTION

The invention is based on the finding of increased levels of circulating IL-18 in patients with acute coronary syndromes and increased IL-18 production in unstable carotid atherosclerotic plaques responsible for stroke. In addition to that, it has been shown that *in vivo* electrotransfer of an expression plasmid DNA encoding for IL-18BP prevents fatty streak development in the thoracic aorta and slows progression of advanced atherosclerotic plaques in the aortic sinus in a well-established murine model of atherosclerosis. More importantly, transfection with the IL-18BP plasmid induces profound changes in plaque composition (decrease in macrophage, T cell, cell death and lipid content and increase in smooth muscle cell and collagen content) leading to a stable plaque phenotype. These results demonstrate for the first time an important role for IL-18 inhibitors in reduction of plaque development/progression and in promotion of plaque stability.

The invention therefore relates to the use of an IL-18 inhibitor as defined in the claims for the manufacture of a medicament for treatment and/or prevention of atherosclerosis.

The term "prevention" within the context of this invention refers not only to a complete prevention of a certain effect, but also to any partial or substantial prevention, attenuation, reduction, decrease or diminishing of the effect before or at early onset of disease.

The term "treatment" within the context of this invention refers to any beneficial effect on progression of disease, including attenuation, reduction, decrease or diminishing of the pathological development after onset of disease.

The term "inhibitor of IL-18" within the context of this invention refers to any molecule modulating IL-18 production and/or action in such a way that IL-18 production and/or action is attenuated, reduced, or partially, substantially or completely prevented or blocked.

An inhibitor of production can be any molecule negatively affecting the synthesis, processing or maturation of IL-18. The inhibitors considered according to the invention can be, for example, suppressors of gene expression of the interleukin IL-18, antisense mRNAs reducing or preventing the transcription of the IL-18 mRNA or leading to degradation of the mRNA, proteins impairing correct folding, or partially or substantively preventing maturation or secretion of IL-18, proteases degrading the IL-18, once it has been synthesized, and the like. An inhibitor of production could be a Caspase-1 inhibitor or an ICE inhibitor, for example, preventing the maturation of IL-18.

An inhibitor of IL-18 action can be an IL-18 antagonist, for example. Antagonists can either bind to or sequester the IL-18 molecule itself with sufficient affinity and specificity to partially or substantially neutralize the IL-18 or IL-18 binding site(s) responsible for IL-18 binding to its ligands (like, e.g. to its receptors). An antagonist may also inhibit the IL-18 signaling pathway, activated within the cells upon IL-18/receptor binding.

Inhibitors of IL-18 action may be also soluble IL-18 receptors or molecules mimicking the receptors, or agents blocking the IL-18 receptors, IL-18 antibodies, like monoclonal antibodies, for example, or any other agent or molecule preventing the binding of IL-18 to its targets, thus diminishing or preventing triggering of the intra- or extracellular reactions mediated by IL-18.

Atherosclerosis is also called arteriosclerosis or hardening of the arteries. Within the context of the present invention, the term atherosclerosis encompasses all diseases or diseased conditions of arteries usually described as atherosclerosis, in which fatty material is deposited in the vessel wall, eventually resulting in narrowing and impairment of blood flow as well as rupture and/or erosion with thrombus formation.

In accordance with the present invention, atherosclerosis is meant to comprise both sclerosis or loss of elasticity of arteries due to atheroma (atherosclerosis) and due to any other cause (arteriosclerosis). The pathological conditions of atherosclerosis, as well as the complications or consequences of atherosclerosis, which are intended to be included in the term "atherosclerosis" as used herein, have been described in detail in the "background of the invention" above.

Progression of atherosclerosis includes formation of atherosclerotic plaques and their development into more and more instable forms. The invention therefore also relates to the use of an IL-18 inhibitor for the manufacture of a medicament for reducing or preventing the progression of atherosclerosis.

Vessel occlusion by a thrombus formed on an atherosclerotic plaque is the critical event in infarctions of the heart and the brain, which are among the most harmful consequences of atherosclerosis. Therefore, the invention also relates to the use of an IL-18 inhibitor for the manufacture of a medicament for treatment and/or prevention of thrombosis of an atherosclerotic plaque.

Plaque stability influences the development of an atherosclerotic plaque into a harmful or vulnerable plaque, which is prone to initiate thrombosis. Therefore, the invention further relates to the use of an IL-18 inhibitor for the manufacture of a medicament for prevention and/or treatment of atherosclerotic plaque instability.

An unstable plaque is prone to disruption, and disruption of a plaque may lead to thrombosis. The invention therefore further relates-to the use of an IL-18 inhibitor for the manufacture of a medicament for prevention of atherosclerotic plaque erosion or disruption.

The plaque instability and thrombosis may e.g. be due to apoptotic cell death, which confers high procoagulant activity and might be a key event leading to thrombosis of eroded or ruptured atherosclerotic plaques as well as embolic events (13, 14). It has been shown that oxidized lipoproteins (oxLDL) induce macrophage and endothelial cell apoptosis in culture (15). As shown in the examples below, is has now been found that an inhibitor of IL-18 is capable of greatly reducing the cell death induced by oxLDL.

In accordance with the present invention, it has been surprisingly found that IL-18 levels in the blood were significantly elevated in heart failure patients suffering form recurrent events, like e.g. death, recurrent ischemia, re-vascularisation, progression of atherosclerosis or re-hospitalization for heart failure, as compared to the patients who did not return to hospital. This increase in IL-18 levels was especially pronounced in those patients who died later, as compared to the ones who survived. Elevated IL-18 levels in the blood circulation were observed in both ischemia patients, as well as in non-ischemic patients.

In a preferred embodiment of the invention, the IL-18 inhibitor is selected from the group consisting of antibodies against IL-18, antibodies against any of the IL-18 receptor subunits, and IL-18 binding proteins, muteins of IL-18BP comprising conservative amino acid substitutions, fused proteins of IL-18BP and immunoglobulin heavy chain regions, functional derivatives comprising PEGylated IL-18BP, wherein the mutein fused protein or functional derivative retains the biological activity of binding to IL-18,

As used herein the term "muteins" refers to analogs of an IL-18BP, in which one or more of the amino acid residues or a natural IL-18BP are replaced by different amino acid residues selected from what are known as "conservative" substitutions. Conservative amino acid substitutions of IL-18BP polypeptides or proteins or viral IL-18BPs, may include synonymous amino acids within a group which have sufficiently similar physicochemical properties that substitution between members of the group will preserve the biological function of the molecule (16). It is clear that insertions and deletions of amino acids may also be made in the above-defined sequences without altering their function, particularly if the insertions or deletions only involve a few amino acids, e.g., under thirty, and preferably under ten, and do not remove or displace amino acids which are critical to a functional conformation, e.g., cysteine residues. Proteins and muteins produced by such deletions and/or insertions come within the purview of the present invention.

Preferably, the synonymous amino acid groups are those defined in Table I. More preferably, the synonymous amino acid groups are those defined in Table II; and most preferably the synonymous amino acid groups are those defined in Table III.

**TABLE I**

| **Preferred Groups of Synonymous Amino Acids** | |
|---|---|
| **Amino Acid** | **Synonymous Group** |
| Ser | Ser, Thr, Gly, Asn |
| Arg | Arg, Gln, Lys, Glu, His |
| Leu | lie, Phe, Tyr, Met, Val, Leu |
| Pro | Gly, Ala, Thr, Pro |
| Thr | Pro, Ser, Ala, Gly, His, Gin, Thr |
| Ala | Gly, Thr, Pro, Ala |
| Val | Met, Tyr, Phe, lie, Leu, Val |
| Gly | Ala, Thr, Pro, Ser, Gly |
| Ile | Met, Tyr, Phe, Val, Leu, lie |
| Phe | Trp, Met, Tyr, lie, Val, Leu, Phe |
| Tyr | Trp, Met, Phe, Ile, Val, Leu, Tyr |
| Cys | Ser, Thr, Cys |
| His | Glu, Lys, Gin, Thr, Arg, His |
| Gln | Glu, Lys, Asn, His, Thr, Arg, Gln |
| Asn | Gln, Asp, Ser, Asn |
| Lys | Glu, Gln, His, Arg, Lys |
| Asp | Glu, Asn, Asp |
| Glu | Asp, Lys, Asn, Gin, His, Arg, Glu |
| Met | Phe, Ile, Val, Leu, Met |
| Trp | Trp |

**TABLE II**

| **More Preferred Groups of Synonymous Amino Acids** | |
|---|---|
| **Amino Acid** | **Synonymous Group** |
| Ser | Ser |
| Arg | His, Lys, Arg |
| Leu | Leu, Ile, Phe, Met |
| Pro | Ala, Pro |
| Thr | Thr |
| Ala | Pro, Ala |
| Val | Val, Met, Ile |
| Gly | Gly |
| Ile | Ile, Met, Phe, Val, Leu |
| Phe | Met, Tyr, Ile, Leu, Phe |
| Tyr | Phe, Tyr |
| Cys | Cys, Ser |
| His | His, Gin, Arg |
| Gln | Glu, Gln, His |
| Asn | Asp, Asn |
| Lys | Lys, Arg |
| Asp | Asp, Asn |
| Glu | Glu, Gin |
| Met | Met, Phe, Ile, Val, Leu |
| Trp | Trp |

**TABLE III**

| **Most Preferred Groups of Synonymous Amino Acids** | |
|---|---|
| **Amino Acid** | **Synonymous Group** |
| Ser | Ser |
| Arg | Arg |
| Leu | Leu, lie, Met |
| Pro | Pro |
| Thr | Thr |
| Ala | Ala |
| Val | Val |
| Gly | Gly |
| Ile | Ile, Met, Leu |
| Phe | Phe |
| Tyr | Tyr |
| Cys | Cys, Ser |
| His | His |
| Gln | Gln |
| Asn | Asn |
| Lys | Lys |
| Asp | Asp |
| Glu | Glu |
| Met | Met, Ile, Leu |
| Trp | Met |

Examples of production of amino acid substitutions in proteins which can be used for obtaining muteins of IL-18BP polypeptides or proteins for use in the present invention include any known method steps, such as presented in US patents 4,959,314, 4,588,585 and 4,737,462, to Mark et al; 5,116,943 toKoths et al., 4,965,195 to Namen et al; 4,879,111 to Chong et al; and 5,017,691 to Lee et al; and lysine substituted proteins presented in US patent No. 4,904,584 (Shaw et al).

The term "fused protein" refers to a polypeptide comprising an IL-18BP fused with another protein, which, *e.g.*, has an extended residence time in body fluids. An IL-18BP may thus be fused to an immunoglobulin or a fragment thereof.

"Functional derivatives" as used herein cover derivatives of IL-18BPs including polyethylene glycol side-chains, which may mask antigenic sites and extend the residence of an IL-18BP in body fluids.

In a further preferred embodiment of the invention, the inhibitor of IL-18 is an IL-18 antibody. Anti-IL-18 antibodies may be polyclonal or monoclonal, chimeric, humanised; or even fully human. Recombinant antibodies and fragments thereof are characterised by high affinity binding to IL-18 *in vivo* and low toxicity. The antibodies which can be used in the invention are characterised by their ability to treat patients for a period sufficient to have good to excellent regression or alleviation of the pathogenic condition or any symptom or group of symptoms related to a pathogenic condition, and a low toxicity.

Neutralising antibodies are readily raised in animals such as rabbits, goat or mice by immunisation with IL-18. Immunised mice are particularly useful for providing sources of B cells for the manufacture ofhybridomas, which in tum are cultured to produce large quantities of anti-IL-1 8 monoclonal antibodies.

Chimeric antibodies are immunoglobulin molecules characterised by two or more segments or portions derived from different animal species. Generally, the variable region of the chimeric antibody is derived from a non-human mammalian antibody, such as murine monoclonal antibody, and the immunoglobulin constant region is derived from a human immunoglobulin molecule. Preferably, both regions and the combination have low immunogenicity as routinely determined (24). Humanised antibodies are immunoglobulin molecules created by genetic engineering techniques in which the murine constant regions are replaced with human counterparts while retaining the murine antigen binding regions. The resulting mouse-human chimeric antibody preferably have reduced immunogenicity and improved pharmacokinetics in humans (25).

Thus, in a further preferred embodiment, IL-18 antibody is a humanised IL-18 antibody. Preferred examples of humanized anti-IL-18 antibodies are described in the European Patent Application EP 0 974 600, for example.

In yet a further preferred embodiment, the IL-18 antibody is fully human. The technology for producing human antibodies is described In detail e.g. in WO00/76310, WO99/53049, US 6,162,963 or AU 5336100. Fully human antibodies are preferably recombinant antibodies, produced in transgenic animals, e.g. xenomice, comprising all or parts of functional human Ig loci.

In a highly preferred embodiment of the present invention, the inhibitor of IL-18 Is a IL-18BP a mutein of IL-18BP comprising conservative amino acid substitutions, fused protein of IL-18BP and immunoglobulin heavy claim regions, functional derivative comprising PEGylated IL-18BP. These muteins, fused proteins or functional derivatives retain the biological activity of IL-18BP, namely the binding to IL-18, and preferably have essentially at least an activity similar to IL-18BP. Ideally, such proteins have a biological activity which is even increased in comparison to unmodified IL-18BP.

The sequences of IL-18BP and its splice variants/isoforms can be taken from WO99/09063 or from (12), as well as from (23).

Functional derivatives of IL-18BP are conjugated to polymers in order to improve the properties of the protein, such as the stability, half-life, bioavailability, tolerance by the human body, or immunogenicity. To achieve this goal, IL18-BP is linked to Polyethlyenglycol (PEG). PEGylation may be carried out by known methods, described in WO 92/13095, for example.

Therefore, in a preferred embodiment of the present invention, IL-18BP is PEGylated.

The invention also refers to a fused protein comprising IL-18 binding protein, which is fused part of an immunoglobulin. The person skilled in the art will understand that the resulting fusion protein retains the biological activity of IL-18BP, in particular the binding to IL-18. The fusion may be direct, or via a short linker peptide which can be as short as 1 to 3/amino acid residues in length or longer, for example, 13 amino acid residues in length. Said linker may be a tripeptide of the sequence E-F-M (Glu-Phe-Met), for example, or a 1,3-amino add linker sequence comprising Glu-Phe-Gly-Ala-Gly-Leu-Val-Leu-Gly-Gly-Gln-Phe-Met introduced between the IL-18BP sequence and the immunoglobulin sequence. The resulting fusion protein has improved properties, such as an extended residence time in body fluids (hatf-life), increased specific activity, increased expression level, or the purification of the fusion protein is facilitated.

IL-18BP is fused to heavy chain regions, like the CH2 and CH3 domains of human IgG1, for example. The generation of specific fusion proteins comprising IL-18BP and a portion of an immunoglobulin are described in example 11 of WO99/09063, for example. Other isoforms of Ig molecules are also suitable for the generation of fusion proteins according to the present invention, such as isoforms IgG₂ or IgG₄, or other lg classes, like IgM or IgA, for example. Fusion proteins may be monomeric or multimeric, hetero- or homomultimeric.

Interferons are predominantly known for inhibitory effects on viral replication and cellular proliferation. Interferon-γ, for example, plays an important role in promoting immune and inflammatory responses. Interferon β (IFN-β, an interferon type I), is said to play an anti-inflammatory role.

The invention also relates to the use of a combination of an inhibitor of IL-18 and an interferon in the manufacture of a medicament for the treatment of atherosclerosis.

Interferons may also be conjugated to polymers in order to improve the stability of the proteins. A conjugate between Interferon β and the polyol Polyethlyenglycol (PEG) has been described in WO99/55377, for instance.

In another preferred embodiment of the invention, the interferon is Interferon-β (IFN-β), and more preferably IFN-β 1a.

The inhibitor of IL-18 production and/or action is preferably used simultaneously, sequentially, or separately with the interferon.

In yet a further embodiment of the invention, an inhibitor of IL-18 is used in combination with a TNF antagonist. TNF antagonists exert their activity in several ways. First, antagonists can bind to or sequester the TNF molecule itself with sufficient affinity and specificity to partially or substantially neutralise the TNF epitope or epitopes responsible for TNF receptor binding (hereinafter termed "sequestering antagonists"). A sequestering antagonist may be, for example, an antibody directed against TNF.

Alternatively, TNF antagonists can inhibit the TNFsignalling pathway activated by the cell surface receptor after TNF binding (hereinafter termed "signalling antagonists"). Both groups of antagonists are useful, either alone or together, in combination with an IL-18 inhibitor, in the therapy of atherosclerosis.

TNF antagonists are easily identified and evaluated by routine screening of candidates for their effect on the activity of native TNF on susceptible cell lines in vitro, for example human B cells, in which TNF causes proliferation and immunoglobulin secretion. The assay contains TNF formulation at varying dilutions of candidate antagonist, e.g. from 0,1 to 100 times the molar amount of TNF used in the assay, and controls with no TNF or only antagonist (26).

Sequestering antagonists are the preferred TNF antagonists to be used according to the present invention. Amongst sequestering antagonists, those polypeptides that bind TNF with high affinity and possess low immunogenicity are preferred. Soluble TNF receptor molecules and neutralising antibodies to TNF are particularly preferred. For example, soluble TNF-RI and TNF-RII are useful in the present invention. Truncated forms of these receptors, comprising the extracellular domains of the receptors or functional portions thereof, are more particularly preferred antagonists according to the present invention. Truncated soluble TNF type-I and type-II receptors are described in EP914431, for example.

Truncated forms of the TNF receptors are soluble and have been detected in urine and serum as 30 kDa and 40 kDa TNF inhibitory binding proteins, which are called TBPI and TBPII, respectively (27). The simultaneous, sequential, or separate use of the IL-18 inhibitor with the TNF antagonist and /or an Interferon is preferred, according to the invention.

According to the invention, TBP I and TBPII are preferred TNF antagonists to be used in combination with an IL-18 inhibitor. Derivatives, fragments, regions and biologically active portions of the receptor molecules functionally resemble the receptor molecules that can also be used in the present invention. Such biologically active equivalent or derivative of the receptor molecule refers to the portion of the polypeptide, or of the sequence encoding the receptor molecule, that is of sufficient size and able to bind TNF with such an affinity that the interaction with the membrane-bound TNF receptor is inhibited or blocked.

In a further preferred embodiment, human soluble TNF-RI (TBPI) is the TNF antagonist to be used according to the invention. The natural and recombinant soluble TNF receptor molecules and methods of their production have been described in the European Patents EP 308 378, EP 398 327 and EP 433 900.

The IL-18 inhibitor can be used simultaneously, sequentially or separately with the TNF inhibitor. Advantageously, a combination of an IL-18 antibody or antiserum and a soluble receptor of TNF, having TNF inhibiting activity, is used.

In a further preferred embodiment of the invention, the medicament further comprises a COX-inhibitor, preferably a COX-2 inhibitor. COXinhibitors are known in the art. Specific COX-2 inhibitors are disclosed in WO 01/00229, for example.

Inhibitors of thromboxane, in particular thromboxane A2, are presently widely used for the treatment of atherosclerosis. Therefore, in a further preferred embodiment of the invention, the medicament further comprises a thromboxane inhibitor, and in particular an inhibitor of thromboxane A2, for simultaneous, sequential or separate use. Aspirin is especially preferred to be used in combination with the IL-18 inhibitor, according to the invention.

One of the causes of atherosclerosis seems to be a high concentration of lipids in the blood. Therefore, in a further preferred embodiment, the medicament further comprises a lipid lowering agent for simultaneous, sequential or separate use. Any lipid lowering agent known in the art may be used according to the invention, such as Further fat lowering agents comprise medications such as cholestyramine, colestipol, nicotinic acid, gemfibrozil, probucol, and others. Especially preferred are HMG CoA Reductase inhibitors, and preferably the so-called statins. Many statins are known in the art, such as Simvastatin or lovastatin.

In order to prevent and/or treat atherosclerosis even better, a preferred embodiment of the invention pertains to the use of an IL-18 inhibitor in combination with a low-fat and/or low-cholesterol and/or low-salt diet.

In a preferred embodiment of the present invention, the inhibitor of IL-18 is used in an amount of about 0.0001 to 10 mg/kg of body weight, or about 0.01 to 5 mg/kg of body weight or about 0.1 to 3 mg/kg of body weight or about 1 to 2 mg/kg of body weight. In yet a further preferred embodiment, the inhibitor of IL-18 is used in an amount of about 0.1 to 1000 µg/kg of body weight or 1 to 100 µg/kg of body weight or about 10 to 50 µg/kg of body weight.

The invention further relates to the as defined in the claims use of an expression vector comprising the coding sequence of an inhibitor of IL-18 in the preparation of a medicament for the prevention and/or treatment of atherosclerosis. A gene therapeutical approach is thus used for treating and/or preventing the disease. Advantageously, the expression of the IL-18 inhibitor will then be *in situ,* thus efficiently blocking IL-18 directly in the tissue(s) or cells affected by the disease.

As explained in detail in the examples below, it has been shown that an efficient expression of IL-18BP could be shown in a murine model of disease after electrotransfer of an expression vector comprising the IL-18BP coding sequence.

Therefore, in a preferred embodiment, the expression vector is administered by electrotransfer, preferably intramuscularly.

The use of a vector for inducing and/or enhancing the endogenous production of an inhibitor of IL-18 as defined in the claims in a cell normally silent for expression of the IL-18 inhibitor, or which expresses amounts of the inhibitor which are not sufficient, are also contemplated according to the invention. The vector may comprise regulatory sequences functional in the cells desired to express the inhibitor or IL-18. Such regulatory sequences may be promoters or enhancers, for example. The regulatory sequence may then be introduced into the right locus of the genome by homologous recombination, thus operably linking the regulatory sequence with the gene, the expression of whichis required to be induced or enhanced. The technology is usually referred to as "endogenous gene activation" (EGA), and it is described e.g. in WO 91/09955.

It will be understood by the person skilled in the art that it is also possible to shut down IL-18 expression using the same technique, i.e. by introducing a negative regulation element, like e.g. a silencing element, into the gene locus of IL-18, thus leading to down-regulation or prevention of IL-18 expression. The person skilled in the art will understand that such down-regulatlon or silencing of IL-18 expression has the same effect as the use of an IL-18 inhibitor in order to prevent and/or treat disease.

The invention further relates to the use of a cell that has been genetically modified to produce an inhibitor of IL-18 as defined in the claims in the manufacture of a medicament for the treatment and/or prevention of atherosclerosis.

The inhibitors of IL-18BP as defined in the claims can be formulated into pharmaceutical compositions, particularly useful for prevention and/or treatment of atherosclerosis, which comprise a therapeutically effective amount of an inhibitor of the IL-18 and a therapeutically effective amount of an interferon. As Inhibitor of IL-18, the composition may comprise an inhibitor as claimed.

IL-18BP and muteins of IL-18BP comprising conservative amino acid substitutions, fused proteins of IL-18BP and immunoglobulin heavy chain regions or, functional derivatives comprising PEGylated IL-18BP, wherein the mutein fused protein, or functional derivative retains the biological activity of binding to IL-18, are the preferred active ingredients of the pharmaceutical compositions.

The interferon comprised in the pharmaceutical composition is preferably IFN-β.

In yet another preferred embodiment, the pharmaceutical composition comprises therapeutically effective amounts of an IL-18 inhibitor according to the claims, optionally an interferon, and a TNF antagonist The TNF antagonists may be antibodies neutralising TNF activity, or soluble truncated TNF receptor fragments, also called TBPI and TPBII. The pharmaceutical composition according to the invention may further comprise one or more COX Inhibitors, preferably COX-2 inhibitors. The pharmaceutical composition according to the invention may further comprise a thromboxane Inhibitor, such as aspirin, and/or a lipid-lowering agent such as a statin.

The definition of pharmaceutically acceptable" is meant to encompass any carrier, which does not interfere with effectiveness of the biological activity of the active ingredient and that is not toxic to the host to which it is administered. For example, for parenteral administration, the active protein(s) may be formulated in a unit dosage form for injection in vehicles such as saline, dextrose solution, serum albumin and Ringer's solution.

The active ingredients of the pharmaceutical composition according to the invention can be administered to an individual in a variety of ways. The routes of administration include intradermal, transdermal (e.g. in slow release formulations), intramuscular, intraperitoneal, Intravenous, subcutaneous, oral, epidural, topical, and intranasal routes. Any other therapeutically efficacious route of administration can be used, for example absorption through epithelial or endothelial tissues or by gene therapy wherein a DNA molecule encoding the active agent is administered to the patient (e.g. via a vector) which causes the active agent to be expressed and secreted in vivo. In addition, the protein(s) according to the invention can be administered together with other components of biologically active agents such as pharmaceutically acceptable surfactants, excipients, carriers, diluents and vehicles.

For parenteral (e.g. intravenous, subcutaneous, intramuscular) administration, the active protein(s) can be formulated as a solution, suspension, emulsion or lyophilised powder In association with a pharmaceutically acceptable parenteral vehicle (e.g. water, saline, dextrose solution) and additives that maintain isotonicity (e.g. mannitol) or chemical stability (e.g. preservatives and buffers). The formulation is sterilized by commonly used techniques.

The bioavailability of the active protein(s) according to the invention can also be ameliorated by using conjugation procedures which increase the half-life of the molecule in the human body, for example linking the molecule topolyethylenglycol, as described in the PCT Patent Application WO 92/13095.

The therapeutically effective amounts of the active protein(s) will be a function of many variables, including the type of antagonist, the affinity of the antagonist for IL-18, any residual cytotoxic activity exhibited by the antagonists, the route of administration, the clinical condition of the patient (including the desirability of maintaining a non-toxic level of endogenous IL-18 activity

A "therapeutically effective amount" is such that when administered, the IL-18 inhibitor results in inhibition of the biological activity of IL-18. The dosage administered, as single or multiple doses, to an individual will vary depending upon a variety of factors, including IL-18 inhibitor pharmacokinetic properties, the route of administration, patient conditions and characteristics (sex, age, body weight, health, size), extent of symptoms, concurrent treatments, frequency of treatment and the effect desired. Adjustment and manipulation of established dosage ranges are well within the ability of those skilled in the art, as well as in vitro and in vivo methods of determining the inhibition of IL-18 in an individual.

According to the invention, the inhibitor of IL-18 is used in an amount of about 0.0001 to 10 mg/kg or about 0.01 to 5 mg/kg or body weight, or about 0.01 to 5 mg/kg of body weight or about 0.1 to 3 mg/kg of body weight or about 1 to 2 mg/kg of body weight. Further preferred amounts of the IL-18 inhibitors are amounts of about 0.1 to 1000µg/kg of body weight or about 1 to 100 µg/kg of body weight or about 10 to 50 µg/kg of body weight

The route of administration which is preferred according to the invention is administration by subcutaneous route. Intramuscular administration is further preferred according to the invention.

In further preferred embodiments, the inhibitor of IL-18 is administered daily or every other day.

The daily doses are usually given in divided doses or in sustained release form effective to obtain the desired results. Second or subsequent administrations can be performed at a dosage which is the same, less than or greater than the initial or previous dose administered to the individual. A second or subsequent administration can be administered during or prior to onset of the disease.

According to the invention, the IL-18 inhibitor can be administered prophylactically or therapeutically to an individual prior to, simultaneously or sequentially with other therapeutic regimens or agents (e.g. multiple drug regimens), in a therapeutically effective amount. Active agents that are administered simultaneously with other therapeutic agents can be administered in the same or different compositions.

The invention further relates to the use of IL-18 as a diagnostic marker for progression of atherosclerosis.

Having now described the invention, it will be more readily understood by reference to the following examples that are provided by way of illustration and are not intended to be limiting of the present invention.

### EXAMPLES

### Material and methods

### Specimens

Forty one human atherosclerotic plaques removed from 36 patients undergoing carotid endarterectomy were collected. For controls, 2 carotid and 3 internal mammary arteries free of atherosclerosis (2 with minimal fibromuscular thickening) were obtained at autopsy or during coronary bypass surgery. They were rapidly immersed in liquid nitrogen and stored at -80°C. Plaques that were used for protein and RNA extraction were rapidly washed, immersed in liquid nitrogen before they were stored at -80°C. For immunohistochemical studies, plaques were placed for 2 hours in fresh 4% paraformaldehyde, then transferred to a 30% sucrose-PBS solution before being snap-frozen in optimal cutting temperature tissue processing medium (O.C.T. Compound, Miles Inc, Diagnostics Division) with liquid nitrogen and stored at -80°C for cryostat sectioning. Several 8- to 10-µm sections were obtained from each specimen for histological analysis and immunohistochemical studies.

### Patient classification

In order to study the potential relation between IL-18/IL-18BP expression and signs of plaque instability, we collected in a prospective and blinded manner clinical data from 23 consecutive patients (out of 36) undergoing the endarterectomy procedure between May and August 2000. The presence or absence of an intra-plaque ulcer on macroscopic examination was systematically reported by the surgeon who performed the endarterectomy procedure. This enabled us to classify the plaques as ulcerated or non-ulcerated plaques. In addition, the patients were classified according to clinical symptoms in two separate groups. Patients who presented with clinical symptoms of cerebral ischemic attack related to the carotid stenosis were classified as symptomatic. Endarterectomy was performed 2-66 days (17.6 ± 5.3 days) after the onset of clinical symptoms in these patients. Patients who never experienced symptoms of cerebral ischemia in the carotid artery territory were classified as asymptomatic. Asymptomatic carotid stenosis was detected on the basis of systematic clinical examination of patients with coronary or peripheral disease, and its severity was determined using repeated Doppler echography by an experienced validated echographist. Eventhough asymptomatic patients never had an ischemic episode in the territory of the carotid stenosis, carotid endarterectomy has been shown to be beneficial in these patients, as shown by Asymptomatic Carotid Atherosclerosis Study (ACAS) investigators (28).

### Western Blot Analysis

Proteins were extracted from 12 atherosclerotic plaques and 5 control normal arteries. Frozen samples were pulverized under liquid nitrogen. The powders were resuspended in ice-cold lysis buffer [20 mmol/L Tris-HCl, pH 7.5, 5 mmol/L EGTA, 150 mmol/L NaCl, 20 mmol/L glycerophosphate, 10 mmol/L NaF, 1 mmol/L sodium orthovanadate. 1% Triton X-100, 0.1% Tween 20, 1 µg/mL aprotinin, 1 mmol/L PMSF, 0.5 mmol/L N-tosyl-L-phenylalanine chloromethyl ketone (TPCK), 0.5 mmol/L N(a)-p-tosyl-L-lysine chloromethyl ketone (TLCK)] at a ratio of 0.3 mL/10 mg of wet weight. Extracts were incubated on ice for 15 minutes and then centrifuged (12 000 g, 15 minutes, 4°C). The detergent-soluble supernatant fractions were retained, and protein concentrations in samples were equalised by using a Bio-Rad protein assay.

In order to perform western blot assays for IL-18 and IL-18R, protein extracts were boiled for 5 minutes and loaded on a 7.5% or 15% SDS-polyacrylamide gel. For IL-18BP, rhlL-1 8 purified from E.Coli (Serono Pharmaceutical Research Institute, Geneva) was coupled to Affligel 15 (Biorad) at 1mg/ml of resin according to the manufacturer's protocol. Protein extract (60 µg) were incubated overnight at 4°C on a roller with 20 µl of resin adjusted to 500 µl of PBS 0.05% Tween. In order to remove any non-specific binding, the resin was centrifuged and washed with 10 mM Tris pH 8, 140 mM NaCl, 0.5% Triton-X-100 (Fluka), 0.5% deoxycholate, then with 50 mM Tris pH 8, 200 mM NaCl, 0.05% TX100, 0.05% nonidet P40. (Fluka), 2 mM CHAPS (Boehringer, Mannheim) followed by a last wash with 50 mM Tris pH 8. The resin was then centrifuged, resuspended in sample buffer under reduced conditions, boiled for 5 minutes and finally loaded on a 10% SDS-polyacrilamide NuPAGE gel (Invitrogen).

Samples were electrophoretically transferred from polyacrylamide gels onto nitrocellulose. Nitrocellulose membranes were saturated for 2 hours at room temperature in TBST [50 mmol/L Tris-HCl (pH 7.5), 250 mmol/L NaCl, and 0.1% Tween saline] containing 5% of fat-free dry milk. Membranes were then incubated with goat anti-human IL-18 and IL-18R (α-chain) polyclonal antibodies (1 µg/ml) (R & D Systems), mouse anti-human IL-18 BP monoclonal antibody (Mab 657.27 at 5 µg/ml) (Corbaz et al., 2000 manuscript submitted), rabbit anti-human caspase-1 polyclonal antibody (1 µg/ml) (A-19, Santa Cruz). The specificity of Mab 657.27 was analysed on stripped membrane by competition using a 200 x molar excess of rhIL-18BP-6his (purified fromchinese hamster ovary cells, Serono Pharmaceutical Research Institute) coincubated with the Mab 657.27 at 5 µg/ml for 1 h. Following incubation with HRP conjugated corresponding antibodies, chemiluminescence substrates (ECL, Western blotting; Amersham Corp) were used to reveal positive bands according to the manufacturer's instructions, and bands were visualised after exposure to Hyperfilm ECL (Amersham Corp).

### Immunohistochemistry

Frozen sections from 6 atherosclerotic plaques were incubated with 1:10 normal horse serum or 1:10 normal goat serum for 30 minutes at room temperature, washed once in PBS, then incubated with either a primary mouse monoclonal antibody against CD68 for macrophage identification (DAKO-CD68, KP1), or a primary mouse monoclonal antibody against human smooth muscle α-actin (1A4, DAKO) for identification of smooth muscle cells. To identify IL-18 and IL-18 receptor within atherosclerotic plaques, specific goat polyclonal antibodies (R & D Systems) were used at a dilution of 5 µg/mL. IL-18 BP was detected by use of a specific monoclonal antibody directed against recombinant human IL-18BP isoform a (H20) (Corbaz et al., 2000 manuscript submitted). After washing in PBS, the slides were incubated with the following secondary biotinylated antibodies: a biotinylated horse anti-mouse IgG (Vector Laboratories, Inc) at a dilution of 1:200 for detection of stains with antibodies against CD68, smooth muscle α-actin and IL-18 BP, and a biotinylated horse anti-goat IgG (Vector) at a dilution of 1:200 for detection of anti-IL-18 and anti-IL-18 receptor antibodies. Immunostains were visualised with the use of avidin-biotin HRP visualisation systems (Vectastain ABC kit PK-6100 Vector). For negative controls, adjacent sections were stained with isotype-matched irrelevant antibodies instead of the primary antibodies.

### RNA preparation

Total RNA was extracted from 29 atherosclerotic plaques in an acid guanidium thiocyanate solution and extracted with phenol and chloroform according to the method of Chomczynski and Sacchi (29). The purified RNA was dissolved in water and the concentration measured by absorbance at 260 nm. RNA integrity was assessed by electrophoresis on 1 % agarose gels. cDNA was synthesised from 1µg of total RNA using the Promega reverse transcription system according to the manufacturer's protocol.

### Semi-quantitative and Real-time PCR of human IL-18 and IL-18BP in human atherosclerotic plaques.

Semi-quantitative PCR reactions were performed in a total volume of 50 µl in the presence of 1 U of AmpliTaq DNA Polymerase (Perkin Elmer, Roche, U.S.A), 2.5 mM dNTPs (Amersham, U.S.A), and 50 pmoles of forward and reverse PCR primers. Reactions were incubated in a PTC-200 Peltier Effect Thermal Cycler (MJ Research, U.S.A) under the following conditions: denaturation 1 min at 94°C, annealing for 1 min at 55°C and extension for 1 min at 72°C. To ensure to compare the amount of PCR products during the linear phase of the PCR reaction, IL-18BP, IL-18 and β-actin were analysed after 25, 28 and 31 cycles. The optimal number of cycles for IL-18BP, IL-18 and β-actin before saturation of the bands was determined (31, 28 and 25, respectively). PCR primers were designed based on the published sequences (AF110799, D49950, X00351) as follows: IL-18, reverse 5'-GCGTCACTACACTCAGCTAA-3'; forward 5'-GCCTAGAGGTATGGCTGTAA-3'; IL18BP, forward 5'-ACCTGTCTACCTGGAGTGAA-3'; reverse 5'-GCACGAAGATAGGAAGTCTG-3'; β-actin, reverse 5'-GGAGGAGCAATGATCTTGATCTTC-3'; forward 5'-GCTCACCATGGATGATGATATCGC-3'. To exclude the amplification of potential genomic DNA contaminating the samples, PCR reactions were performed in the absence of the cDNA template. PCR products (10µl) were analysed on 1% agarose gels electrophoresed in 1 × TAE buffer. The size of PCR products was verified by comparison with a 1 kb ladder (Gibco) following staining of the gels. Relative quantification of ethidium-bromide stained bands was performed under UV light using the Kodak Digital Sciences analytical software, and was reported as the ratio of target gene (hlL-18BP, hIL-18) to the housekeeping gene (hβ-actin).

SYBR Green Real Time PCR primers for IL-18, IL-18BP and GAPDH (housekeeping control) were designed using the Primer Express software from PE Biosystems according to the published sequences (AF110799, D49950, NM 002046) as follows: IL-18, reverse 5'-CAGCCGCTTTAGCAGCCA-3'; forward 5'-CAAGGAATTGTCTCCCAGTGC-3'; IL18BP, reverse 5'-AACCAGGCTTGAGCGTTCC-3'; forward 5'-TCCCATGTCTCTGCTCATTTAGTC-3'; GAPDH, reverse 5'-GATGGGATTTCCATTGATGACA-3'; forward 5'-CCACCCATGGCAAATTCC-3'; intron-GAPDH, reverse 5'-CCTAGTCCCAGGGCTTTGATT-3'; forward 5'-CTGTGCTCCCACTCCTGATTTC-3'. The specificity and the optimal primer concentration were tested. Potential genomic DNA contamination was excluded by performing PCR reactions with specific intron-GAPDH primers. The absence of non-specific amplification was confirmed by analysing the PCR products by a 3.5% agarose gel electrophoresis. SYBR Green Real-Time PCR was performed with 5 µl / well of RT-products (0.5 ng total RNA), 25 µl / well of SYBR Green PCR master mix (PE Biosystem, CA, USA) with AmpErase Uracil N-Glycosylase (UNG) (0.5 Unit / well) and 20 µl of primers (300 nM). PCR was performed at 50°C for 2 min (forAmpErase UNG incubation to remove any uracil incorporated into the cDNA), 95°C for 10 min (for AmpliTaq Gold activation) and then run for 40 cycles at 95°C for 15 sec, 60°C for 1 min on the ABI PRISM 7700 Detection System. The reverse-transcribed cDNA samples were thus amplified and their Ct (cycle threshold) values were determined. All Ct values were normalised to the housekeeping gene GAPDH. A single specific DNA band for IL-18, IL-18BP and GAPDH was observed using gel electrophoresis analysis.

The principle of real-time detection using the "SYBR Green PCR master mix" is based upon the direct detection of PCR product by measuring the increase in fluorescence caused by the binding of SYBR Green dye to double-stranded DNA.

### Statistical analysis

Data are expressed as mean ± SEM. Levels of IL-18 were compared between groups using the Mann-Whitney test. A value of p 0.05 was considered statistically significant.

### Example 1: Protection by IL-18 inhibitors from endothelial cell death induced by oxidised lipoproteins (oxLDL)

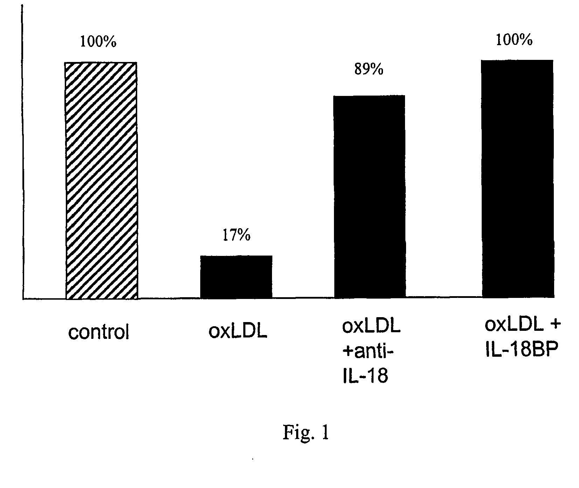

Cultured human umbilical vein endothelial cells (HUVECs) were exposed for 16 hours to oxLDL in the presence or absence of IL-18 binding protein or anti-IL-18 antibody. As shown in Fig. 1, 83% of HUVECs died after exposure to oxLDL. The co-incubation with IL-18BP or anti-IL-18 antibody almost totally rescued the cells from death. No death was observed using IL-18BP. 89% of the cells survived using the anti-IL-18 antibody.

This experiment clearly shows the protective effect of two different IL-18 inhibitors against cells death due to apoptosis within the atherosclerotic plaque.

### Example 2: Expression of IL-18 protein and its endogenous inhibitor IL-18 BP in atherosclerotic plaques

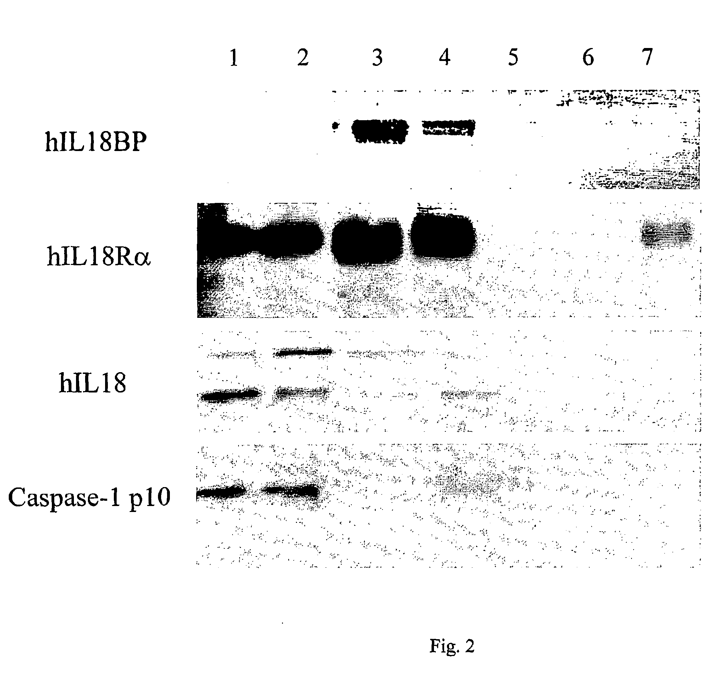

Western blot assays were performed on protein extracts from 12 carotid atherosclerotic arteries and 5 normal controls. IL-18 protein, including the active form, was highly expressed in all atherosclerotic plaques whereas little or no expression was detected in normal arteries (Fig. 2). Lanes 1 to 4 contain samples from atherosclerotic plaques, lanes 5 to 7 from normal arteries. Interestingly, detection of the active form of IL-18 seemed to correlate with the expression of the active form of caspase-1, which is involved in IL-18 processing (Fig. 2 forth row). Significant expression of IL-18 receptor protein (the α chain) was also detected in all atherosclerotic plaques in comparison with a very low level of expression in normal arteries (Fig. 2 second row). In addition, a majority of atherosclerotic plaques expressed IL-18BP although the level of expression was heterogeneous (Fig. 2 first row).

### Example 3: Cellular localisation of IL-18 protein and its endogenous inhibitor IL-18BP in atherosclerotic plaques

In order to determine the cellular localisation of IL-18 and IL-18BP, immunohistochemical studies were performed on 6 carotid atherosclerotic plaques. As shown in figure 2, IL-18 was mainly expressed in macrophages, these cells being probably the major source of IL-18 in the plaque (not shown). These areas were also rich in CD3-positive lymphocytes. However, T lymphocytes did not seem to be directly involved in IL-18 production. IL-18 was also expressed in some intimal smooth muscle cells and in occasional endothelial cells. In contrast, significant expression of IL-18BP was detected in endothelial cells of plaque microvessels and in those of the luminal surface, although the expression was not found in all vessels. Relatively low and more heterogeneous IL-18 BP expression was also detected, mainly extracellularly, in some macrophage-rich areas.

### Example 4: Expression of IL-18 and IL-18BP mRNA transcripts in atherosclerotic plaques and relation to plaque instability

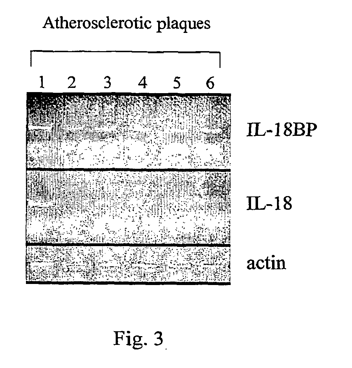

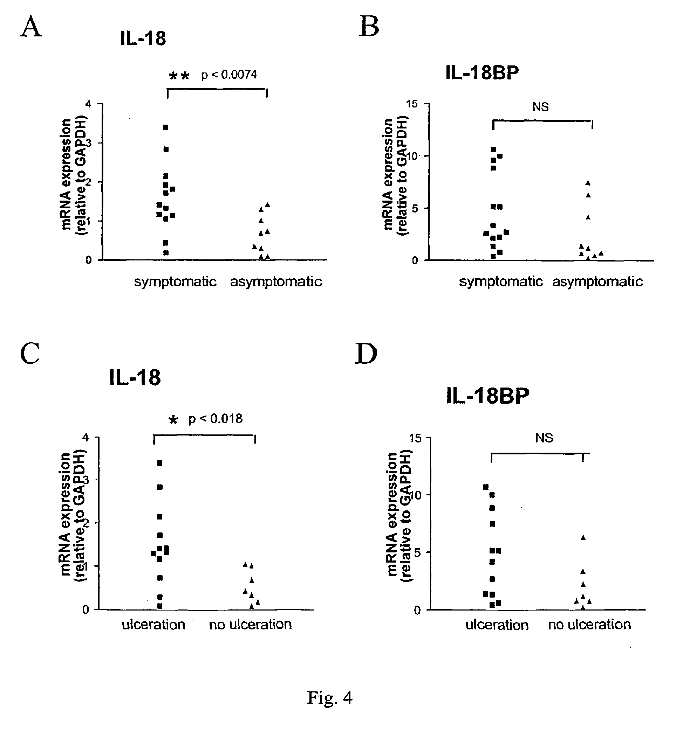

In order to determine whether human IL-18 and IL-18BP mRNA were expressed in human carotid atherosclerotic plaques, semi-quantitative RT-PCR was performed on six atherosclerotic plaques (Fig. 3). IL-18 and IL-18BP mRNA were detected in all atherosclerotic plaques although the amount of mRNA for IL-18 and IL-18BP was heterogeneous. Therefore, in order to accurately quantitate the levels of IL-18 and IL-18BP mRNA expression, 23 atherosclerotic plaques were further analyzed with the SYBR Green Real-Time PCR method (Fig. 4). The plaques were characterized by clinical and pathological examination as symptomatic (unstable) or asymptomatic (stable) plaques, containing macroscopic ulcer or not. The clinical characteristics of the patients are summarised in Table 4. Percentage of carotid diameter reduction (60%-95%) and risk factors, including age, diabetes, hypercholesterolemia, hypertension, and cigarette smoking did not differ between the two groups.

**Table 4**

| Patient characteristics | | |
|---|---|---|
| | Asymptomatic | Symptomatic |
| | Patients (n = 9) | Patients' (n = 14) |
| Age | 66.9 ± 4.0 | 70.2 ± 3.9 |
| Gender | Male (8) | Male (9) |
| Hypertension² | 8 | 9 |
| Hypercholesterolemia³ | 4 | 8 |
| Diabetes | 3 | 1 |
| Currently smoking | 7 | 8 |
| Coronary artery disease | 5 | 4 |

| | | |
|---|---|---|
| ¹ These patients presented with transient or persistent ischemic cerebral attack 2-66 days before endarterectomy. ²Number of patients with clinical hypertension being treated with antihypertensive agents. ³Number of patients with clinical hypercholesterolemia being treated with lipid-lowering drugs. | | |

The amount of IL-18 was found to be upregulated in the symptomatic compared to the asymptomatic atherosclerotic plaques (2.03 ± 0.5 vs 0.67 ± 0.17, respectively). (Fig. 4A). Statistical analysis demonstrated that this increase in IL-18 production observed in the symptomatic plaques was highly significant (p < 0.0074), whereas the increased amount of IL-18BP observed in the symptomatic versus the asymptomatic plaques was not (4.64 ± 0.98 vs 2.5 ± 0.92, respectively) (Fig. 4B). In other terms, although both symptomatic and asymptomatic groups showed positive correlation between IL-18 and IL-18BP mRNA, the slopes were significantly different between the 2 groups (symptomatic group: slope 1.16 [0.19-2.14], r² = 0.36 vs asymptomatic group: slope 4.79 [2.39-7.20], r² = 0.76; p < 0.05). Therefore, it seems that the relative increase in IL-18BP expression in the symptomatic group is not sufficient enough to compensate for the increase in IL-18 expression. Moreover, as the presence of ulceration is considered as a feature of instability in the plaques, statistical analysis was further performed on plaques without or with intra-plaque ulcers and demonstrated a significant upregulation of IL-18 in the plaques presenting ulcers (p < 0.018) (Fig. 4C).

These data show that the increase in IL-18 expression seen in the atherosclerotic plaques correlates with the instability of the plaque.

### Example 5: IL-18BP modulates atherosclerotic lesion development and stability in an in vivo model of disease

### Methods

### Patients characteristics

Plasma samples were obtained from patients with acute ischemic coronary syndromes (unstable angina and myocardial infarction), less than 7 days following the initiation of symptoms. Unstable angina was defined as the association of typical chest pain with either ischemic changes on the electrocardiogram or the presence of coronary artery disease. Myocardial infarction was diagnosed on the basis of typical ischemic changes on the electrocardiogram associated with significant increases in myocardial enzymes (creatine phosphokinase and troponin I) in the circulating blood. Non-ischemic patients were recruited in the same cardiology department and were completely free of ischemic signs. Plasma levels of human IL-18 were determined using a commercially available kit (MBL, Japan).

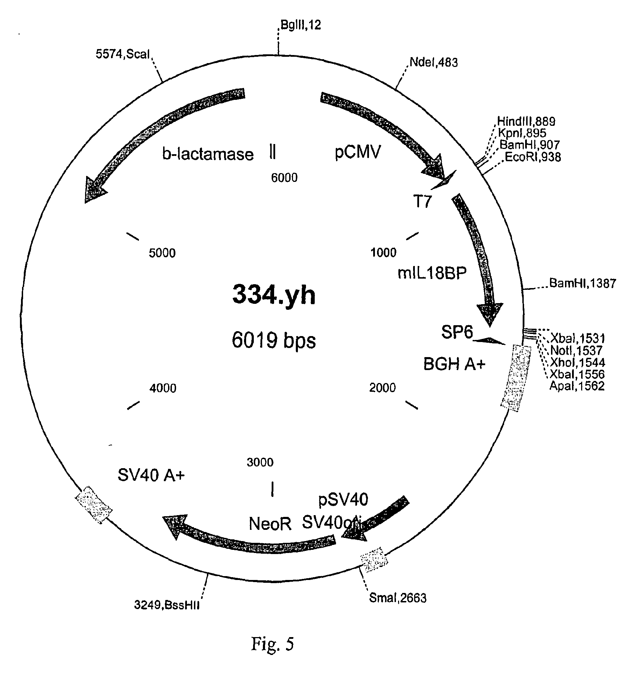

*In vivo intramuscular electrotransfer of murine IL-18SP expression plasmid* Fourteen male C57BU6 apoE KO mice, 14-week-old, received at 3-week-interval, 3 injections with the IL-18BP expression plasmid, pcDNA3-IL18BP. The control mice (n = 19) were injected with the control empty plasmid. Murine IL-18BP isoform d cDNA isolated as described (accessory number # Q9ZOM9) (23) was subcloned into the EcoR1/Not1 sites of mammalian cell expression vector pcDNA3 under the control of the cytomegalovirus promotor (Invitrogen). The construct, called 334.yh, is shown in Fig. 5. Control plasmid was a similar construct devoid of therapeutic cDNA. The IL-18BP or control expression plasmid (60 µg) was injected in both tibial cranial muscles of the anesthetised mouse as previously described (13). Briefly, transcutaneous electric pulses (8 square wave electric pulses of 200 V/cm, 20 msec duration at 2 Hz) were delivered by a PS-15 electropulsator (Genetronics, France) using two stainless steel plate electrodes placed 4.2 to 5.3 mm apart, at each side of the leg.

### Elisa mIL-18BP

Plates were coated overnight with r-mIL-18BPd-affinity purified rabbit polyclonal antibody (5 µg/well). Soluble mIL-18BP was detected using a biotinylated rabbit polyclonal antibody (0.3 µg/ml) raised against E. coli r-mIL-18BP (Peprotec) followed by extravidin peroxidase (1/1000) (Sigma). The capture rabbit polyclonal antibody was tested by Western Blot in order to confirm mIL-18BP specificity. Recombinant mIL-18BPd produced by HEK 293 cells was used as standard. The sensitivity of the ELISA was 5 ng/ml.

### Analysis of mice

Cryostat sections (8 µm) were obtained from the aortic sinus and were used for detection of lipid deposition using Oil red, detection of collagen using Sirius red and for immunohistochemical analysis as previously described (13). The sections were stained with specific primary antibodies: anti-mouse macrophage, clone MOMA2 (BioSource), phosphatase alkaline-conjugated anti-α-actin for smooth muscle cells and anti-CD3 for T lymphocytes (Dako) as previously described (13). Detection of cell death was performed using the TUNEL technique (13). CD3 positive cells were microscopically counted in a blinded manner. Atherosclerotic plaques in the aortic sinus and areas that stained positive for macrophages, smooth muscle cells, collagen or TUNEL were measured using computer assisted-image quantification (NS15000, Microvision) as previously described (13). Staining with non-immune isotype-matched immunoglobulins assessed specificity of the immunostaining. Specificity of TUNEL was assessed by omission of the enzyme terminal deoxynucleotidyl transferase. The thoracic aortas, spanning from the left subclavian artery to the renal arteries, were fixed with 10% buffered formalin and stained for lipid deposition with Oil red. They were then opened longitudinally and the percentage of lipid deposition was calculated using computer-assisted image quantification (NS15000, Microvision).

### Results

In the present study, the hypothesis was tested hat the IL-18/IL-18BP regulation plays a critical role in both atherogenesis and plaque stability. Plasma levels of IL-18 in patients with acute coronary syndromes (30 males, 18 females, mean age 66.2 ± 1.8 years old, of whom 14 had unstable angina and 34 had myocardial infarction) and in non-ischemic control patients recruited in the same cardiology department (10 males, 3 females, mean age 60.0 ± 5.2 years old) were measured. Plasma levels of IL-18 were significantly elevated in acute coronary patients compared with controls (146.9 ± 17.1 vs 73.0 ± 12.2 pg/ml, respectively, p < 0.05) in contrast to circulating levels of IL-18BP which were slightly increased (20.1 ± 2.7 vs 7.5 ± 2.5 ng/ml, respectively, p = 0.06). In addition, IL-18 levels correlated with the severity of the disease as highest levels were observed in the patients with severe ischemic cardiac dysfunction and clinical signs of pulmonary oedema (224.03 ± 39.1 pg/ml, p < 0.001 compared with controls). These results obtained from patients with acute coronary disease, together with the previous observations that IL-18 is elevated in atherosclerotic plaques from patients with strokes [Mallat, 2001], suggest a potentially important role for the IL-18/IL-18BP regulation in the atherosclerotic process.

We therefore tested this hypothesis using apoE knockout (KO) mice that spontaneously develop human-like atherosclerotic lesions. Fourteen 14-week-old male mice received IL-18BP supplementation through *in vivo* intramuscular electrotransfer of an expression plasmid DNA encoding for murine IL-18BPd, while 19 age-matched controls received the empty plasmid. Plasmid electrotransfer was repeated every 3 weeks and the mice were sacrified at 23 weeks of age following 9 weeks of treatment. Plasma levels of murine IL-18BP were lower than the detection limits (5 ng/ml) in apoE KO mice injected with the empty plasmid. However, a single injection of the IL-18BP plasmid resulted in high levels of IL-18BP in the blood with a maximal risen 2 days after the injection (323.5 ± 100.9 ng/ml) and 127.4 ± 35.4 ng/ml measured after 2 weeks. Following 9 weeks of treatment with either IL-1 8BP or empty plasmid, total cholesterol (489.4 ± 34.6 vs 480.8 ± 36.3 mg/dl, respectively) and high-density lipoprotein serum levels (52.3 ± 9.4 vs 48.8 ± 5.1 mg/dl, respectively) were not different between the 2 groups. A modest but significant increase in animal weight was observed in the IL-18BP-treated group compared to the control-group (31.8 ± 0.9 vs 28.6 ± 0.8 g, respectively, p < 0.05).

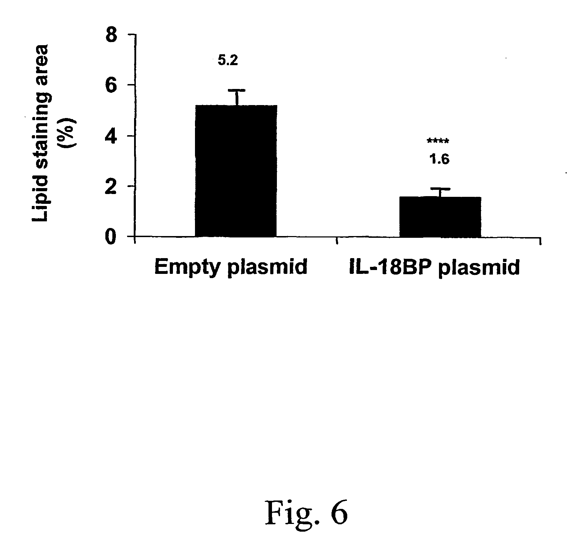

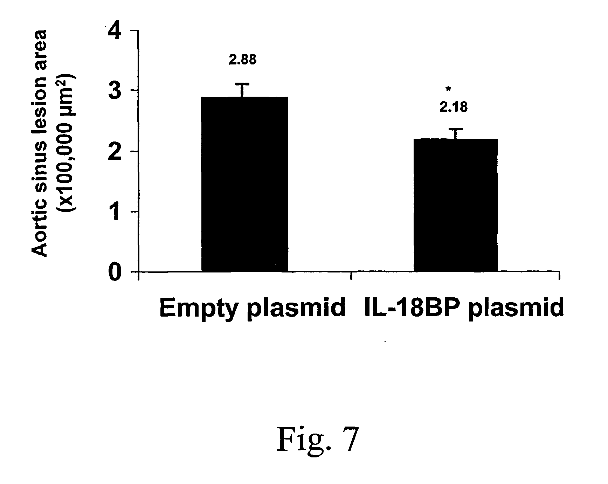

The outcome of IL-18BP supplementation on atherosclerosis was examined in 2 different locations: the descending thoracic aorta and the aortic sinus. The thoracic aorta was chosen to determine the role of IL-18BP in fatty streak development (atherogenesis) since thoracic atherosclerotic lesions are almost absent at the age of 14 weeks (data not shown) where IL-18BP transfection was started. The aortic sinus, where atherosclerotic lesions are already present at 14 weeks of age (data not shown), was examined for advanced plaque progression and composition, an important determinant of plaque stability. IL-18BP-treatment of apoE KO mice significantly affected atherosclerotic lesion development and progression. Examination of the thoracic aorta showed a marked reduction in lipid deposition in mice treated with the IL-18BP plasmid compared to the empty plasmid (Fig. 6). Quantitative computer-assisted image analysis showed 69% reduction in the extent of atherosclerotic lesions (p < 0.0001) (Fig. 6), pointing to a critical permissive role for IL-18 in atherogenesis. In addition, treatment with IL-18BPplasmid for only 9 weeks significantly limited the progression of advanced atherosclerotic plaques in the aortic sinus (24% reduction in plaque size, p = 0.01) compared to treatment with the empty plasmid (Fig. 7).

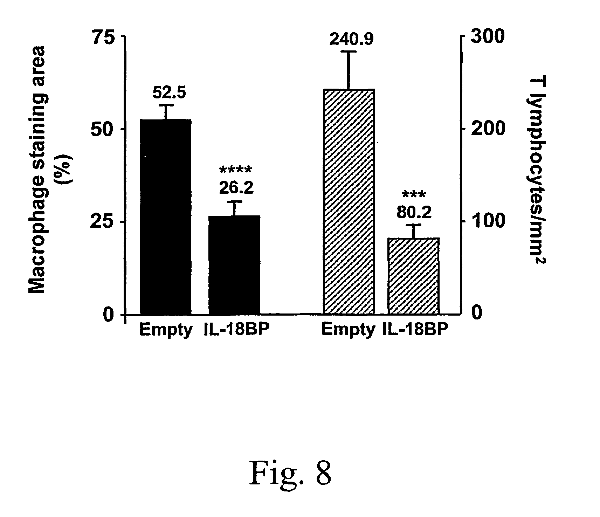

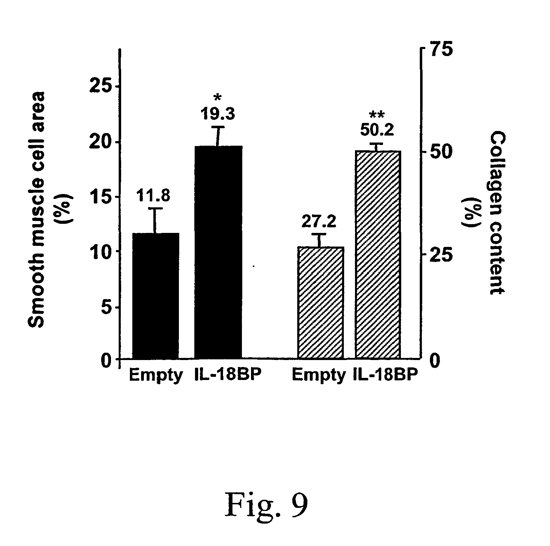

More importantly, the composition of advanced lesions, a major determinant of plaque instability, was profoundly affected by IL-18BP treatment. Atherosclerotic lesions of mice treated with the IL-18BP plasmid exhibited a very significant 50% reduction in macrophage infiltration (p < 0.0001) (Fig. 8), contained 67% fewer T lymphocytes (p < 0.005) (Fig. 8), and showed a 2-fold increase in smooth muscle cell accumulation (p < 0.05) (Fig. 9). In addition, these important changes in lesion cellular composition were associated with a significant 85% increase in collagen content (p < 0.0005) as determined by staining with Sirius red, and a decrease in total lipid content.

Therefore, IL-18BP treatment significantly attenuated the inflammatory process within the atherosclerotic lesions and induced a healing process characteristic of stable atherosclerotic plaques. Furthermore, the marked reduction in the inflammatory component of the lesions in IL-18BP treated mice was associated with a substantial reduction in the occurrence of cell death within the plaques (2.9 ± 0.9% in IL-18BP treated mice vs 10.5 ± 3.6% in controls, p < 0.05), therefore limiting the expansion and thrombogenicity of the acellular lipid core [Mallat, 1999].

### Conclusions:

Using a well-validated mouse model of human-like atherosclerosis, the results reported above clearly establish an unsuspected and crucial role for the IL-18 and IL-18BP regulation in atherosclerotic plaque development, progression and stability. While preventing early lesion formation in the thoracic aorta, inhibition of IL-18 activity by IL-18BP supplementation also profoundly affected advanced lesion composition in the aortic sinus, inducing a switch toward a stable plaque phenotype.

The clinical prognosis of a patient with atherosclerosis depends only in part on the size of the lesions. It is now widely recognised that the quality (plaque composition), rather than the size, of the lesion could be an even better predictor of the occurrence of ischemic events. Indeed, severe clinical manifestations of atherosclerosis (infarctions of the heart and brain) are mainly due to vessel lumen occlusion by a thrombus formed at the contact of a disrupted atherosclerotic plaque (19). Pathological studies have shown that vulnerable or unstable plaques, that are prone to rupture or have ruptured, are rich in inflammatory cells and exhibit a substantial loss in smooth muscle cell and collagen content (20, 21). Moreover, such plaques show significant increase in apoptotic cell death leading to the formation of a highly thrombogenic lipid core (13, 22). It is noteworthy that all these signs of increased plaque instability were markedly attenuated in IL-18BP treated mice, indicating that IL-18 signalling is a major determinant of plaque instability.

The relevance of the results obtained in apoE KO mice to human disease is strengthened by our finding of increased levels of circulating IL-18 in patients with acute coronary syndromes and increased IL-18 production in unstable carotid atherosclerotic plaques responsible for stroke. These findings, taken together, identify inhibitors of IL-18 activity as new important therapeutic tools to prevent and treat atherosclerotic plaque development and to limit plaque complications.

### Example 6: Elevated levels of IL-18 are correlated to recurrent events in heart failure patients

The levels of IL-18 were measured in blood sera of patients by ELSIA with an IL-18 specific antibody.

Altogether, 56 Ischemic or non-ischemic patients, with or without heart failure were tested.

In patients who died later, the levels of IL-18 were 216.0 ± 41.5 pg/ml versus 112.2 ± 12.2 pg/ml in patients without mortal outcome (p = 0.0018).

In patients with any recurrent event, such as death, recurrent ischemia, re-vascularisation, progression of atherosclerosis or re-hospitalization for heart failure, the following IL-18 levels were measured: 165.8 ± 23.8 versus 107.7 ± 14.6 in patients without any recurrent event (p=0.03).

These results demonstrate that IL-18 levels are significantly elevated in patients having a bad clinical prognosis, like recurrent events or even death.

Blood samples in 16 non-ischemic patients, with or without heart failure, were measured for their IL-18 levels. In those patients who died later, the levels were 199.0 ± 34.8 pg/ml versus 95.3 ± 20.4 pg/ml in those patients who survived (p=0.09).

Patients with any recurrent event: 146.6 ± 34.4 pg/ml IL-18 versus 95.4 ± 23.9 pg/ml in patients without recurrent event (p=0.03). Although the differences in IL-18 levels did not reach statistical significance, due to the small number of patients, a clear trend towards elevated levels of IL-18 could be observed.

In ischemic patients, IL-18 levels were 214.2 ± 45.9 pg/ml in the patients who died versus 118.4 + 12.8 pg/ml in those who survived (p=0.007).

162.8 ± 24.7 pg/ml of IL-18 was measured in those patents who had any recurrent event, versus 116.2 ± 16.0 in those without any recurrent events.

### REFERENCES

1. Ross R. Atherosclerosis-an inflammatory disease. N Engl J Med 1999;340:115-126.
2. van der Wal AC, Becker AE, van der Loos CM, Das PK. Site of intimal rupture or erosion of thrombosed coronary atherosclerotic plaques is characterized by an inflammatory process irrespective of the dominant plaque morphology. Circulation 1994;89:36-44.
3. Fuster V, Badimon L, Badimon JJ, Chesebro JH. The pathogenesis of coronary artery disease and the acute coronary syndromes (1). N Engl J Med 1992; 326:242-250.
4. Fuster V, Badimon L, Badimon JJ, Chesebro JH. The pathogenesis of coronary artery disease and the acute coronary syndromes (2). N Engl J Med 1992;326:310-318.
5. Lee RT, Libby P. The unstable atheroma. Arterioscler Thromb Vasc Biol 1997;17:1859-1867.
6. Ridker PM, Cushman M, Stampfer MJ, Tracy RP, Hennekens CH. Inflammation, aspirin, and the risk of cardiovascular disease in apparently healthy men. N Engl J Med 1997;336:973-979.
7. Libby P. Molecular bases of the acute coronary syndromes. Circulation 1995;91:2844-2850.
8. Nakamura, K, Okamura, H, Nagata, K and Tamura, T. (1989). Infect. Immun. 57, 590-595.
9. Yoshimoto T, Takeda, K, Tanaka, T, Ohkusu, K, Kashiwamura, S, Okamura, H, Akira, S and Nakanishi, K (1998). J. Immunol. 161, 3400-3407.
10. Parnet, P, Garka, K E, Bonnert, T P, Dower, S K, and Sims, J E. (1996). J. Biol. Chem. 271, 3967-3970.
11. DiDonato, J A, Hayakawa, M, Rothwarf, D M, Zandi, E and Karin, M. (1997). Nature 388, 16514-16517.
12. Novick, D, Kim, S-H, Fantuzzi, G, Reznikov, L, Dinarello, C, and Rubinstein, M (1999). Immunity 10,127-136.
13. Mallat Z, Hugel B, Ohan J, Lesèche G, Freyssinet JM, Tedgui A. Shed membrane microparticles with procoagulant potential in human atherosclerotic plaques. A role for apoptosis in plaque thrombogenicity. Circulation 1999;99:348-353.
14. Tricot O, Mallat Z, Heymes C, Belmin J, Lesèche G, Tedgui A. Relation Between Endothelial Cell Apoptosis and Blood Flow Direction in Human Atherosclerotic Plaque.Circulation 2000;in press.
15. Dimmeler S, Haendeler J, Galle J, Zeiher AM. Oxidized low-density lipoprotein induces apoptosis of human endothelial cells by activation of CPP32-like proteases. A mechanistic due to the 'response to injury' hypothesis. Circulation 1997;95:1760-3.
16. Grantham, Science, Vol. 185, pp. 862-864 (1974).
17. Dimmeler S, Haendeler J, Galle J, Zeiher AM. Oxidized low-density lipoprotein induces apoptosis of human endothelial cells by activation of CPP32-like proteases. A mechanistic due to the 'response to injury' hypothesis. Circulation 1997;95:1760-3.
18. Mir LM. Bureau MF, Gehl J, Rangara R, Rouy D, Caillaud JM, Dellere P, Branellec D, Schwartz B, Scherman D. High efficiency gene transfer into skeletal muscle mediated by electric pulses. Proc Natl Acad Sci USA 1999; 96:4262-7.
19. Lee, R.T. & Libby, P. The unstable atheroma. Arterioscler Thromb Vasc Biol 17,1859-1867 (1997).
20. Davies, M.J. The composition of coronary-artery plaques [editorial; comment]. N Engl J Med 336, 1312-1314 (1997).
21. Virmani, R., Kolodgie, F.D., Burke, A.P., Farb, A. & Schwartz, S.M. Lessons from sudden coronary death: a comprehensive morphological classification scheme for atherosclerotic lesions. Arterioscler Thromb Vasc Biol 20, 1262-1275. (2000).
22. Kolodgie, F.D. et al. Localization of apoptotic macrophages at the site of plaque rupture in sudden coronary death [In Process Citation]. Am J Pathol 157, 1259-1268 (2000).
23. Kim, S.H. et al. Structural requirements of six naturally occurring isoforms of the IL- 18 binding protein to inhibit IL-18. Proc Natl Acad Sci U S A 97, 1190-1195 (2000).
24. Elliott, M.J., Maini, R.N., Feldmann, M., Long-Fox, A., Charles, P., Bijl, H., and Woody, J.N., 1994, Lancet 344, 1125-1127.
25. Knight DM, Trinh H, Le J, Siegel S, Shealy D, McDonough M, Scallon B, Moore MA, Vilcek J, Daddona P, et al. Construction and initial characterization of a mouse-human chimeric anti-TNF antibody.Mol Immunol 1993 Nov 30:16 1443-53
26. Tucci, A., James, H., Chicheportiche, R., Bonnefoy, J.Y., Dayer, J.M., and Zubler, R.H., 1992, J.Immunol. 148, 2778-2784.
27. Engelmann, H., D. Novick, and D. Wallach. 1990. Two tumor necrosis factor-binding proteins purified from human urine. Evidence for immunological cross-reactivity with cell surface tumor necrosis factor receptors. J. Biol. Chem. 265:1531-1536.
28. Moore WS, Barnett HJ, Beebe HG, et al. Guidelines for carotid endarterectomy. A multidisciplinary consensus statement from the Ad Hoc Committee, American Heart Association. Circulation 1995;91:566-79.
29. Chomczynski P, Sacchi N. Single-step method of RNA isolation by acid guanidium thiocyanate-phenol-chloroform extraction. Anal Biochem 1987;162:156-9.

## Claims

1. Use of an IL-18 inhibitor for the manufacture of a medicament for the treatment and/or prevention of atherosclerosis, wherein the IL-18 inhibitor is selected from the group consisting of antibodies against IL-18, antibodies against any of the IL-18 receptor subunits and IL-18 binding protein (IL-18BP), a mutein of IL-18BP comprising conservative amino acid substitutions, a fused protein of IL-18BP and immunolglobulin heavy chain regions, or PEGylated IL-18BP, wherein the mutein, fused protein or PEGylated IL-18BP retains the biological activity of binding to IL-18.

2. Use according to claim 1, wherein the medicament is for the treatment and/or prevention of thrombosis of an atherosclerotic plaque.

3. Use according to claim 1 or 2, wherein the medicament is for the treatment and/or prevention of atherosclerotic plaque ulcer.

4. Use according to any of the preceding claims, wherein the medicament is for the treatment and/or prevention of atherosclerotic plaque destabilisation.

5. Use according to any of the preceding claims, wherein the medicament is for the prevention and/or treatment of ischemic syndromes due to plaque destabilisation.

6. Use according to any of the preceding claims, wherein the medicament is for the treatment and/or prevention of atherosclerotic plaque disruption.

7. The use according to any of the preceding claims, wherein the IL-18 inhibitor is an antibody directed against IL-18.

8. The use according to any of the preceding claims, wherein the antibody is a humanised antibody.

9. The use according to any of the preceding claims, wherein the antibody is a human antibody.

10. The use according to any of claims 1 to 6, wherein the IL-18 inhibitor is IL-18BP, a mutein of IL-18BP comprising conservative amino acid substitutions, a fused protein of IL-18BP and immunolglobulin heavy chain regions, or PEGylated IL-18BP, wherein the mutein, fused protein or PEGylated IL-18BP retains the biological activity of binding to IL-18.

11. The use according to claim 10, wherein the immunoglobulin in the fused protein is of the IgG1 or IgG2 isotype.

12. The use according to any of the preceding claims, wherein the medicament further comprises an interferon for simultaneous, sequential, or separate use.

13. The use according to claim 12, wherein the interferon is interteron-β.

14. The use according to any of the preceding claims, wherein the medicament further comprises a Tumor Necrosis Factor (TNF) antagonist for simultaneous, sequential, or separate use.

15. The use according to claim 14, wherein the TNF antagonist is TBPI and/or TBPII.

16. The use according to any of the preceding claims, wherein the medicament further comprises a COX-inhibitor for simultaneous, sequential, or separate use.

17. The use according to claim 16, wherein the COX-inhibitor is a COX-2 inhibitor.

18. The use according to any of the preceding claims, wherein the medicament further comprises a thromboxane inhibitor, for simultaneous, sequential, or separate use.

19. The use according to claim 18, wherein the thromboxane inhibitor is aspirin.

20. The use according to any of the preceding claims, wherein the medicament further comprises a lipid lowering agent, for simultaneous, sequential, or separate use.

21. The use according to claim 20, wherein the lipid lowering agent is a HMG CoA inhibitor.

22. The use according to claim 21, wherein the HMG CoA inhibitor is a statin.

23. Use according to any of the preceding claims, wherein the medicament is used in combination with low-fat and/or low-cholesterol and/or low-salt diet.

24. The use according to any of the preceding claims, wherein the inhibitor of IL-18 is to be used in an amount of about 0.0001 to 10 mg/kg of body weight, or about 0.01 to 5 mg/kg of body weight or about 0.1 to 3 mg/kg of body weight or about 1 to 2 mg/kg of body weight.

25. The use according to any of the preceding claims, wherein the inhibitor of IL-18 is to be used in an amount of about 0.1 to 1000 µg/kg of body weight or 1 to 100 µg/kg of body weight or about 10 to 50 µg/kg of body weight.

26. The use according to any of the preceding claims, wherein the IL-18 inhibitor is to be administered subcutaneously.

27. The use according to any of the preceding claims, wherein the IL-18 inhibitor is to be administered intramuscularly.

28. The use according to any of the preceding claims, wherein the IL-18 inhibitor is to be administered daily.

29. The use according to any of the preceding claims, wherein the IL-18 inhibitor is to be administered every other day.

30. Use of an expression vector comprising the coding sequence for an IL-18 inhibitor for the manufacture of a medicament for the treatment and/or prevention of atherosclerosis, wherein the IL-1.8 inhibitor is selected from the group consisting of antibodies against IL-18, antibodies against any of the IL-18 receptor subunits and an IL-18BP, a mutein of IL-18BP comprising conservative amino acid substitutions, a fused protein of IL-18BP and immunolglobulin heavy chain regions, or PEGylated IL-18BP, wherein the mutein, fused protein or PEGylated IL-18BP retains the biological activity of binding to IL-18.

31. Use according to claim 30, wherein the expression vector is administered by electrotransfer.

32. Use according to claim 30 or 31, wherein the expression vector is administered systemically.

33. Use according to any of the preceding claims, wherein the expression vector is administered intramuscularly.

34. Use of a cell that has been genetically modified to produce an inhibitor of IL-18 in the manufacture of a medicament for the treatment and/or prevention of atherosclerosis, wherein the IL-18 inhibitor is selected from the group consisting of antibodies against IL-18, antibodies against any of the IL-18 receptor subunits, and IL-18BP, a mutein of IL-18BP comprising conservative amino acid substitutions, a fused protein of IL-18BP and immunolglobulin heavy chain regions, or PEGylated IL-18BP, wherein the mutein, fused protein or PEGylated IL-18BP retains the biological activity of binding to IL-18.

35. Use of IL-18 as a diagnostic marker of progression of atherosclerosis.

## Patentansprüche

1. Verwendung eines lL-1 8-Inhibitors zur Herstellung eines Medikaments zur Behandlung und/oder Verhinderung von Atherosklerose, worin der IL-18-Inhibitor ausgewählt wird aus der Gruppe, bestehend aus Antikörpern gegen IL-18, Antikörpern gegen eine beliebige der IL-18-Rezeptor-Untereinheiten und IL-18-Bindungsprotein (IL-18BP), einem Mutein von IL-18BP, umfassend konservative Aminosäuresubstitutionen, ein fusioniertes Protein von IL-18BP und Schwere Kette-Regionen von Immunglobulin, oder PEGyliertes IL-18BP, worin das Mutein, fusionierte Protein oder PEGylierte IL-18BP die biologische Aktivität der Bindung an IL-18 beibehält.

2. Verwendung nach Anspruch 1, worin das Medikament zur Behandlung und/oder Verhinderung von Thrombose von einem atherosklerotischen Plaque dient.

3. Verwendung nach Anspruch 1 oder 2, worin das Medikament zur Behandlung und/oder Verhinderung von atherosklerotischem Plaque-Ulkus dient.

4. Verwendung nach einem der vorhergehenden Ansprüche, worin das Medikament zur Behandlung und/oder Verhinderung der Destabilisierung von atherosklerotischem Plaque dient.

5. Verwendung nach einem der vorhergehenden Ansprüche, worin das Medikament zur Behandlung und/oder Verhinderung von ischämischen Syndromen aufgrund von Plaquedestabilisierung dient.

6. Verwendung nach einem der vorhergehenden Ansprüche, worin das Medikament zur Behandlung und/oder Verhinderung von Disruption von atherosklerotischem Plaque dient.

7. Verwendung nach einem der vorhergehenden Ansprüche, worin der IL-18-Inhibitor ein gegen IL-18 gerichteter Antikörper ist.

8. Verwendung nach einem der vorhergehenden Ansprüche, worin der Antikörper ein humanisierter Antikörper ist.

9. Verwendung nach einem der vorhergehenden Ansprüche, worin der Antikörper ein humaner Antikörper ist.

10. Verwendung nach einem der Ansprüche 1 bis 6, worin der IL-18-Inhibitor IL-18-BP, ein Mutein von IL-18BP, umfassend konservative Aminosäuresubstitutionen, ein fusioniertes Protein von IL-18 und Immunglobulin-schwere Kette-Regionen, oder PEGyliertes IL-18BP, worin das Mutein, fusionierte Protein oder PEGylierte IL-18BP die biologische Aktivität der Bindung an IL-18 beibehält, ist.

11. Verwendung nach Anspruch 10, worin das Immunglobulin in dem fusionierten Protein vom IgG1- oder IgG2-Isotyp ist.

12. Verwendung nach einem der vorhergehenden Ansprüche, worin das Medikament weiterhin ein Interferon zur gleichzeitigen, sequenziellen oder getrennten Anwendung umfasst.

13. Verwendung nach Anspruch 12, worin das Interferon β-Interferon ist.

14. Verwendung nach einem der vorhergehenden Ansprüche, worin das Medikament weiterhin einen Tumor-Nekrose-Faktor (TNF)-Antagonisten zur gleichzeitigen, sequenziellen oder getrennten Anwendung umfasst.

15. Verwendung nach Anspruch 14, worin der TNF-Antagonist TBPI und/oder TBPII ist.

16. Verwendung nach einem der vorhergehenden Ansprüche, worin das Medikament weiterhin einen COX-Inhibitor zur gleichzeitigen, sequenziellen oder getrennten Anwendung umfasst.

17. Verwendung nach Anspruch 16, worin der COX-Inhibitor ein COX-2-Inhibitor ist.

18. Verwendung nach einem der vorhergehenden Ansprüche, worin das Medikament weiterhin einen Thromboxaninhibitor zur gleichzeitigen, sequenziellen oder getrennten Anwendung umfasst.

19. Verwendung nach Anspruch 18, worin der Thromboxaninhibitor Aspirin ist.

20. Verwendung nach einem der vorhergehenden Ansprüche, worin das Medikament weiterhin ein Lipid-Verringerungsmittel zur gleichzeitigen, sequenziellen oder getrennten Anwendung umfasst.

21. Verwendung nach Anspruch 20, worin das Lipid-Verringerungsmittel ein HMG CoA-Inhibitor ist.

22. Verwendung nach Anspruch 21, worin der HMG CoA-Inhibitor ein Statin ist.

23. Verwendung nach einem der vorhergehenden Ansprüche, worin das Medikament in Kombination mit einer Diät mit geringem Fett und/oder geringem Cholesterol und/oder geringem Salz verwendet wird.

24. Verwendung nach einem der vorhergehenden Ansprüche, worin der Inhibitor von IL-18 in einer Menge von etwa 0,0001 bis 10 mg/kg Körpergewicht oder etwa 0,01 bis 5 mg/kg Körpergewicht oder etwa 0,1 bis 3 mg/kg Körpergewicht oder etwa 1 bis 2 mg/kg Körpergewicht zu verwenden ist.

25. Verwendung nach einem der vorhergehenden Ansprüche, worin der Inhibitor von IL-18 in einer Menge von etwa 0,1 bis 1000 µg/kg Körpergewicht oder 1 bis 100 µg/kg Körpergewicht oder etwa 10 bis 50 µg/kg Körpergewicht zu verwenden ist.

26. Verwendung nach einem der vorhergehenden Ansprüche, worin der IL-18-Inhibitor subkutan zu verabreichen ist.

27. Verwendung nach einem der vorhergehenden Ansprüche, worin der IL-18-Inhibitor intramuskulär zu verabreichen ist.

28. Verwendung nach einem der vorhergehenden Ansprüche, worin der IL-18-Inhibitor täglich zu verabreichen ist.

29. Verwendung nach einem der vorhergehenden Ansprüche, worin der IL-18-Inhibitor jeden zweiten Tag zu verabreichen ist.

30. Verwendung eines Expressionsvektors, umfassend die codierende Sequenz für einen IL-18-Inhibitor zur Herstellung eines Medikaments zur Behandlung und/oder Verhinderung von Atherosklerose, worin der IL-18-Inhibitor ausgewählt wird aus der Gruppe, bestehend aus Antikörpern gegen IL-18, Antikörpern gegen eine beliebige der IL-18-Rezeptor-Untereinheiten und einem IL-18BP, einem Mutein von IL-18BP, umfassend konservative Aminosäuresubstitutionen, ein fusioniertes Protein von IL-18BP und Schwere Kette-Regionen von Immunglobulin, oder PEGyliertes IL-18BP, worin das Mutein, fusionierte Protein oder PEGylierte IL-18BP die biologische Aktivität der Bindung an IL-18 beibehält.

31. Verwendung nach Anspruch 30, worin der Expressionsvektor durch Elektrotransfer verabreicht wird.

32. Verwendung nach Anspruch 30 oder 31, worin der Expressionsvektor systemisch verabreicht wird.

33. Verwendung nach einem der vorhergehenden Ansprüche, worin der Expressionsvektor intramuskulär verabreicht wird.

34. Verwendung einer Zelle, die genetisch modifiziert worden ist zum Produzieren eines Inhibitors von IL-18 bei der Herstellung eines Medikaments zur Behandlung und/oder Verhinderung von Atherosklerose, worin der IL-18-Inhibitor ausgewählt wird aus der Gruppe, bestehend aus Antikörpern gegen IL-18, Antikörpern gegen eine beliebige der IL-18-Rezeptor-Untereinheiten und IL-18-BP, einem Mutein von IL-18BP, umfassend konservative Aminosäuresubstitutionen, ein fusioniertes Protein von IL-18BP und Schwere Kette-Regionen von Immunglobulin, oder PEGyliertes IL-18BP, worin das Mutein, fusionierte Protein oder PEGylierte IL-18BP die biologische Aktivität der Bindung an IL-18 beibehält.

35. Verwendung von IL-18 als ein diagnostischer Marker der Progression von Atherosklerose.

## Revendications

1. Utilisation d'un inhibiteur de l'IL-18 pour la préparation d'un médicament pour le traitement et/ou la prévention de l'athérosclérose, dans lequel l'inhibiteur de l'IL-18 est choisi dans l'ensemble comprenant les anticorps contre l'IL-18, les anticorps contre n'importe laquelle des sous-unités du récepteur de l'IL-18 et la protéine de liaison de l'IL-18 (IL-18BP), une mutéine de l'IL-18BP comprenant des substitutions conservatrices d'aminoacides, une protéine condensée de l'IL-18BP et des régions à chaînes lourdes des immunoglobulines, ou l'IL-18BP PEGylée ; la mutéine, la protéine condensée ou l'IL-18BP PEGylée conservant l'activité biologique de liaison à l'IL-18.

2. Utilisation conforme à la revendication 1, dans laquelle le médicament est destiné au traitement et/ou à la prévention de la thrombose d'une plaque athéroscléreuse.

3. Utilisation conforme à la revendication 1 ou 2, dans laquelle le médicament est destiné au traitement et/ou à la prévention d'un ulcère de plaque athéroscléreuse.

4. Utilisation conforme à l'une quelconque des revendications précédentes, dans laquelle le médicament est destiné au traitement et/ou à la prévention de la déstabilisation de la plaque athéroscléreuse.

5. Utilisation conforme à l'une quelconque des revendications précédentes, dans laquelle le médicament est destiné à la prévention et/ou au traitement des syndromes ischémiques dus à la déstabilisation de la plaque.

6. Utilisation conforme à l'une quelconque des revendications précédentes, dans laquelle le médicament est destinée au traitement et/ou à la prévention de la rupture de la plaque athéroscléreuse.

7. Utilisation conforme à l'une quelconque des revendications précédentes, dans laquelle l'inhibiteur de l'IL-18 est un anticorps dirigé contre l'IL-18.

8. Utilisation conforme à l'une quelconque des revendications précédentes, dans laquelle l'anticorps est un anticorps humanisé.

9. Utilisation conforme à l'une quelconque des revendications précédentes, dans laquelle l'anticorps est un anticorps humain.

10. Utilisation conforme à l'une quelconque des revendications 1 à 6, dans laquelle l'inhibiteur de l'IL-18 est l'IL-18BP, une mutéine de l'IL-18BP comprenant des substitutions conservatrices d'aminoacides, une protéine condensée de l'IL-18BP et des régions à chaînes lourdes d'immunoglobulines, ou l'IL-18BP PEGylée ; la mutéine, la protéine condensée ou l'IL-18BP PEGylée conservant l'activité biologique de fixation à l'IL-18.

11. Utilisation conforme à la revendication 10, dans laquelle l'immunoglobuline dans la protéine condensée est de l'isotype IgG1 ou IgG2.

12. Utilisation conforme à l'une quelconque des revendications précédentes, dans laquelle le médicament comprend en outre un interféron pour un emploi simultané, successif ou distinct.

13. Utilisation conforme à la revendication 12, dans laquelle l'interféron est l'interféron β.

14. Utilisation conforme à l'une quelconque des revendications précédentes, dans laquelle le médicament comprend en outre un antagoniste du facteur de nécrose tumorale (TNF) pour un emploi simultané, successif ou distinct.

15. Utilisation conforme à la revendication 14, dans laquelle l'antagoniste du TNF est TBPI et/ou TBPII.

16. Utilisation conforme à l'une quelconque des revendications précédentes, dans laquelle le médicament comprend en outre un inhibiteur de COX pour une utilisation simultanée, successive ou distincte.

17. Utilisation conforme à la revendication 16, dans laquelle l'inhibiteur de COX est un inhibiteur de COX-2.

18. Utilisation conforme à l'une quelconque des revendications précédentes, dans laquelle le médicament comprend en outre un inhibiteur du thromboxane pour une utilisation simultanée, successive ou distincte.

19. Utilisation conforme à la revendication 18, dans laquelle l'inhibiteur du thromboxane est l'aspirine.

20. Utilisation conforme à l'une quelconque des revendications précédentes, dans laquelle le médicament comprend en outre un agent hypolipémiant pour un emploi simultané, successif ou distinct.

21. Utilisation conforme à la revendication 20, dans laquelle l'agent hypolipémiant est un inhibiteur de la HMG CoA

22. Utilisation conforme à la revendication 21, dans laquelle l'inhibiteur de la HMG CoA est une statine.

23. Utilisation conforme à l'une quelconque des revendications précédentes, dans laquelle le médicament est utilisé en combinaison avec un régime à faible teneur en graisses, et/ou à faible teneur en cholestérol et/ou à faible teneur en sel.

24. Utilisation conforme à l'une quelconque des revendications précédentes, dans laquelle l'inhibiteur de l'IL-18 doit être utilisé en une quantité d'environ 0,0001 à 10 mg/kg de poids corporel, ou d'environ 0,01 à 5 mg/kg de poids corporel, ou d'environ 0,1 à 3 mg/kg de poids corporel ou d'environ 1 à 2 mg/kg de poids corporel.

25. Utilisation conforme à l'une quelconque des revendications précédentes, dans laquelle l'inhibiteur de l'IL-18 doit être utilisé en une quantité d'environ 0,1 à 1000 µg/kg de poids corporel ou de 1 à 100 µg/kg de poids corporel ou d'environ 10 à 50 µg/kg de poids corporel.

26. Utilisation conforme à l'une quelconque des revendications précédentes, dans laquelle l'inhibiteur de l'IL-18 doit être administré par voie sous-cutanée.

27. Utilisation conforme à l'une quelconque des revendications précédentes, dans laquelle l'inhibiteur de l'IL-18 doit être administré par voie intramusculaire.

28. Utilisation conforme à l'une quelconque des revendications précédentes, dans laquelle l'inhibiteur de l'IL-18 doit être administré journalièrement.

29. Utilisation conforme à l'une quelconque des revendications précédentes, dans laquelle l'inhibiteur de l'IL-18 doit être administré tous les deux jours.

30. Utilisation d'un vecteur d'expression qui comprend la séquence codant pour un inhibiteur de l'Il-18 pour la fabrication d'un médicament pour le traitement et/ou la prévention de l'athérosclérose, dans laquelle l'inhibiteur de l'IL-18 est choisi dans l'ensemble comprenant les anticorps contre l'IL-18, les anticorps contre n'importe laquelle des sous-unités du récepteur de l'IL-18 et une IL-18BP, une mutéine de l'IL-18BP comprenant des substitutions conservatrices d'aminoacides, une protéine condensée de l'IL-18BP et des régions à chaînes lourdes d'immunoglobulines ou l'IL-18BP PEGylée ; la mutéine, la protéine condensée ou l'IL-18BP PEGylée conservant l'activité biologique de fixation à l'IL-18.

31. Utilisation conforme à la revendication 30, dans laquelle le vecteur d'expression est administré par électrotransfert.

32. Utilisation conforme à la revendication 30 ou 31, dans laquelle le vecteur d'expression est administré de manière systémique.

33. Utilisation conforme à l'une quelconque des revendications précédentes, dans laquelle le vecteur d'expression est administré par voie intramusculaire.

34. Utilisation d'une cellule qui a été génétiquement modifiée pour produire un inhibiteur de l'I1-18 dans la fabrication d'un médicament pour le traitement et/ou la prévention de l'athérosclérose, dans laquelle l'inhibiteur d'Il-18 est choisi dans l'ensemble comprenant les anticorps contre I1-18, les anticorps contre n'importe laquelle des sous-unités du récepteur d'IL-18, et l'Il-18BP, une mutéine d'Il-18BP comprenant des substitutions conservatrices d'aminoacides, une protéine condensée d'IL-18BP et des régions à chaînes lourdes d'immunoglobulines, ou l'IL-18BP PEGylée ; la mutéine, la protéine condensée ou l'IL-18BP PEGylée conservant l'activité biologique de fixation à l'IL-18.

35. Utilisation de l'IL-18 comme marqueur diagnostique de progression de l'athérosclérose.
